# EUROPEAN PATENT SPECIFICATION

(11) **EP 1 893 623 B1**
(45) Date of publication and mention of the grant of the patent: **20.05.2009**
(21) Application number: 06762287.8
(22) Date of filing: 29.05.2006
(51) Int. Cl.: C07H 15/203

(54) **C-GLYCOSIDE COMPOUNDS AND USE THEREOF FOR DEPIGMENTING THE SKIN**
C-GLYCOSIDVERBINDUNGEN UND VERFAHREN ZUR HAUTDEPIGMENTIERUNG
COMPOSES C-GLYCOSIDES ET LEUR UTILISATION POUR DEPIGMENTER LA PEAU

(30) Priority: 30.05.2005 FR 0551413; 06.06.2005 US 687307 P
(43) Date of publication of application: 05.03.2008
(73) Proprietor: L'Oréal, 75008 Paris (FR)
(72) Inventor: DALKO, Maria, F-91190 Gif S/Yvette (FR); CAVEZZA, Alexandre, F-93320 Pavillon sous Bois (FR)
(74) Representative: Kromer, Christophe
(86) International application number: PCT/EP2006/006338
(87) International publication number: WO 2006/128738

(56) References cited:
- PAN, HEFENG ET AL: "Phenolic extractives from root bark of Picea abies" PHYTOCHEMISTRY ( 1995 ), 39(6), 1423 -8 CODEN: PYTCAS; ISSN: 0031-9422, 1995, XP002392321
- VIC, GABIN ET AL: "Enzyme-catalyzed synthesis of alkyl .beta.-D-glucosides in organic media" TETRAHEDRON LETTERS ( 1992 ), 33(32), 4567 -70 CODEN: TELEAY; ISSN: 0040-4039, 1992, XP002392322
- TOMMASI, NUNZIATINA DE ET AL: "Aryl and triterpenic glycosides from Margyricarpus setosus" PHYTOCHEMISTRY ( 1996 ), 42(1), 163 -7 CODEN: PYTCAS; ISSN: 0031-9422, 1996, XP002392323
- PATENT ABSTRACTS OF JAPAN vol. 1998, no. 03, 27 February 1998 (1998-02-27) -& JP 09 295927 A (YAGI AKIRA), 18 November 1997 (1997-11-18)
- ZHDANOV, YU. A. ET AL: "Reaction of unsubstituted aldoses with p-methoxybenzoylmethylenephosphoran e" ZHURNAL OBSHCHEI KHIMII , 38(5), 1046-8 CODEN: ZOKHA4; ISSN: 0044-460X, 1968, XP009052288 cited in the application
- ZHDANOV, YU. A. ET AL: "Interaction of partially screened aldoses with p- methoxybenzoylmethylenephosphorane" ZHURNAL OBSHCHEI KHIMII , 39(1), 119-22 CODEN: ZOKHA4; ISSN: 0044-460X, 1969, XP009052287 cited in the application

## Description

The present invention relates to a cosmetic composition for depigmenting and/or bleaching the skin, comprising a C-glycoside compound, and also to certain novel C-glycoside compounds.

The colour of human skin depends on different factors and, in particular, the seasons of the year, race and sex, and it is mainly determined by the nature and concentration of melanin produced by the melanocytes. Melanocytes are specialized cells which synthesize melanin by means of specific organelles, the melanosomes. In addition, at different periods in their life, certain individuals develop darker and/or more coloured blemishes on the skin and more especially on the hands, making the skin non-uniform. These blemishes are also due to a large concentration of melanin in the keratinocytes at the skin surface.

It is most particularly sought to use harmless topical depigmenting substances which are of good efficacy, in order to treat regional hyperpigmentations caused by melanocyte hyperactivity, such as idiopathic melasmas, occurring during pregnancy ("pregnancy mask" or chloasma) or during oestro-progestative contraception, localized hyperpigmentations caused by hyperactivity and proliferation of benign melanocytes, such as senile pigmentation marks known as actinic lentigo, accidental hyperpigmentations, possibly due to photosensitization or to post-lesional cicatrization, as well as certain leukodermias, such as vitiligo. For the latter, (in which the cicatrizations can result in a scar which gives the skin a whiter appearance), failing being able to repigment the damaged skin, the regions of residual normal skin are depigmented in order to give the skin as a whole a uniform white complexion.

The mechanism for the formation of skin pigmentation, that is to say the formation of melanin, is particularly complex and schematically involves the following main steps:

**Tyrosine --> Dopa --> Dopaquinone --> Dopachrome --> Melanin**

Tyrosinase (monophenol dihydroxyl phenylalanine: oxygen oxidoreductase EC 1.14.18.1) is the essential enzyme involved in this reaction sequence. It especially catalyzes the reaction for the conversion of tyrosine into dopa (dihydroxyphenylalanine) by virtue of its hydroxylase activity and the reaction for the conversion of dopa into dopaquinone by virtue of its oxidase activity. This tyrosinase acts only when it is in the mature state, under the action of certain biological factors.

A substance is recognized as being depigmenting if it acts directly on the vitality of the epidermal melanocytes in which melanogenesis takes place, and/or if it interferes with one of the steps in the biosynthesis of melanin either by inhibiting one of the enzymes involved in melanogenesis or by becoming intercalated as a structural analogue of one of the chemical compounds in the melanin synthesis chain, whereby this chain may then be blocked and thus ensure the depigmentation.

The substances most commonly used as depigmenting agents are, more particularly, hydroquinone and its derivatives, in particular its ethers such as hydroquinone monomethyl ether and monoethyl ether. Although they have a certain level of efficacy, these compounds are unfortunately not free of side effects on account of their toxicity, which can make them difficult or even hazardous to use. This toxicity arises from the fact that they interfere with fundamental mechanisms of melanogenesis, by killing cells which then risk disrupting their biological environment and which consequently force the skin to eliminate them by producing toxins.

Thus, hydroquinone is a compound which is particularly irritant and cytotoxic to melanocytes, and whose total or partial replacement has been envisaged by many authors.

Substances have thus been sought which are not involved in the mechanism of melanogenesis, but which act upstream on tyrosinase by preventing its activation, and are consequently much less toxic. Kojic acid is commonly used as tyrosinase-activation inhibitor, this acid complexing the copper present in the active site of this enzyme. Unfortunately, this compound is unstable in solution, which somewhat complicates the manufacture of the composition.

There is still a need for a novel agent for bleaching human skin, which is just as effective as the known agents, but which does not have their drawbacks, i.e. which is non-irritant, non-toxic and/or non-allergizing for the skin, while at the same time being stable in a composition, or alternatively which has reinforced action so as to be able to be used in a smaller amount, thus considerably reducing the observed side effects. In this regard, the Applicant has discovered, surprisingly and unexpectedly, that certain C-glycoside compounds have good depigmenting activity, even at low concentration, without showing any cytotoxicity. Document JP-A-09-95927 discloses sugar ester of 2'-(S)-8-C-glycosyl-7-methylaloesol for whitening the skin.

One subject of the invention is, more specifically, a composition intended especially for depigmenting and/or bleaching the skin, comprising, in a physiologically acceptable medium, at least one compound of formula (I) below: 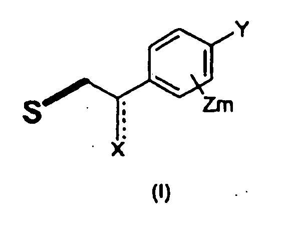 in which:
- S represents a monosaccharide or a polysaccharide containing up to 20 sugar units, in pyranose and/or furanose form and of L and/or D series, the said monosaccharide or polysaccharide containing at least one hydroxyl function that is necessarily free and/or optionally one or more optionally protected amine functions;
- the bond S-CH₂ being a C-glycoside bond;
- X represents a hydrogen atom or a group chosen from -OR₁ and -NR₁R₂ or an =O group;
- Y represents a group chosen from -OR₃, -NR₁R₂ and -OSi (R₄)₃;
- Z represents a fluorine atom or a group chosen from a linear C₁-C₆ alkyl group, a cyclic or branched C₃-C₆ alkyl group, -OR₁, -NR₁R₂, -COR₁, -CO₂R₁, -CONR₁R₂ and -CR₃;
- m is an integer ranging from 0 to 4;
   with
- R₁ and R₂ denoting, independently of each other, a hydrogen atom or a linear C₁-C₆ alkyl group, a cyclic or branched C₃-C₆ alkyl group, aralkyl or phenyl;
- R₃ denoting a hydrogen atom or a linear C₁-C₆ alkyl group, a cyclic or branched C₃-C₆ alkyl group, aralkyl or phenyl, or a group -SO₃H, -PO₃H₂ or -COR₁;
- R₄ denoting a linear C₁-C₆ alkyl group, a cyclic or branched C₃-C₆ alkyl group or phenyl;
and also the cosmetically acceptable salts thereof, solvates thereof such as hydrates.

The compounds of formula (I) according to the invention allow the efficient depigmentation and/or lightening of human skin. They are especially intended to be applied to the skin of individuals with brownish pigmentation marks or senescence marks, or on the skin of individuals wishing to combat the appearance of a brownish colour originating from melanogenesis, for example after an exposure to ultraviolet radiation.

A subject of the invention is thus also a cosmetic process for depigmenting and/or bleaching human skin, comprising the application to the skin of a composition as described above. The process is especially suitable for eliminating brownish pigmentary marks and/or senescence marks, and/or for lightening browned skin.

A subject of the invention is also the cosmetic use of a compound of formula (I) as described above as an agent for bleaching and/or depigmenting the skin, especially for removing pigmentary marks and senescence marks and/or as anti-browning agents.

A subject of the invention is also the use of a compound of formula (I) as described above for the manufacture of a dermatological composition for depigmenting and/or bleaching the skin.

Among the compounds of formula (I), some are known in the prior art.

The document "Separation of α,β-anomers of C-glycosides by medium-pressure liquid chromatography"; Sepu 1988, 6(5), pages 301-3 describes the compounds 2-deoxy-2-(2-(4-methoxyphenyl)ethyl)-D-glucose (CAS 121285-89-0) and 2-deoxy-2-(2-(4-methoxyphenyl)ethyl)-L-glucose (CAS 121285-90-3).

The document "Reaction of unsubstituted aldoses with p-methoxybenzoylmethylenephosphorane"; Zhurnal Obshchei Khimii 1968, 38(5), pages 1046-8 describes the compounds 2-deoxy-2-(2-oxo-2-(4-methoxyphenyl)ethyl)-L-fucose (CAS 20880-40-4), 2-deoxy-2-(2-oxo-2-(4-methoxyphenyl)ethyl)-D-xylose (CAS 20869-22-1) and 2-deoxy-2-(2-oxo-2-(4-methoxyphenyl)ethyl)-L-xylose (CAS 20869-24-3).

The document "Interaction of partially screened aldoses with p-methoxybenzoylmethylenephosphorane"; Zhurnal Obshchei Khimii 1969, 39(1), pages 119-22 describes the compounds 2-deoxy-2-(2-oxo-2-(4-methoxyphenyl)ethyl)-D-maltose (CAS 29461-52-7) and 2-deoxy-2-(2-oxo-2-(4-methoxyphenyl)ethyl)-L-maltose (CAS 24461-51-6).

A subject of the invention is thus also the compounds of formula (I') corresponding to the compounds of formula (I) described above, with the proviso that when m = 0 and Y denotes a -OCH₃ group, S does not denote a D- (or L)-glucose or L-fucose or D- (or L)-xylose or D-(or L)-maltose residue.

In the context of the present invention, the term "alkyl" means a saturated or unsaturated hydrocarbon-based chain. Among the alkyl groups that are suitable for use in the invention, mention may be made especially of methyl, ethyl, isopropyl, n-propyl, n-butyl, t-butyl, isobutyl, sec-butyl, pentyl, n-hexyl, cyclopropyl, cyclopentyl, cyclohexyl and allyl groups.

The acceptable salts for the non-therapeutic use of the compounds described in the present invention include the conventional non-toxic salts of the said compounds, such as those formed from organic or mineral acids. Examples that may be mentioned include the salts of mineral acids, such as sulfuric acid, hydrochloric acid, hydrobromic acid, hydriodic acid, phosphoric acid and boric acid. Mention may also be made of organic acid salts, which may comprise one or more carboxylic, sulfonic or phosphonic acid groups. These may be linear, branched or cyclic aliphatic acids or alternatively aromatic acids. These acids may also comprise one or more heteroatoms chosen from O and N, for example in the form of hydroxyl groups. Mention may be made especially of propionic acid, acetic acid, terephthalic acid, citric acid and tartaric acid.

When the compound of formula (I) comprises an acid group, the acid group(s) may be neutralized with a mineral base, such as LiOH, NaOH, KOH, Ca(OH)₂, NH₄OH Mg(OH)₂ or Zn(OH)₂, or with an organic base such as a primary, secondary or tertiary alkylamine, for example triethylamine or butylamine. This primary, secondary or tertiary alkylamine may comprise one or more nitrogen and/or oxygen atoms and may thus comprise, for example, one or more alcohol functions; mention may be made especially of 2-amino-2-methylpropanol, triethanolamine, dimethylamino-2-propanol and 2-amino-2-(hydroxymethyl)-1,3-propanediol. Mention may also be made of lysine or 3-(dimethylamino)propylamine.

The solvates that are acceptable for the non-therapeutic use of the compounds described in the present invention include conventional solvates such as those formed during the last step in the preparation of the said compounds due to the presence of solvents. Examples that may be mentioned include the solvates due to the presence of water or linear or branched alcohols, for instance ethanol or isopropanol.

One advantageous aspect of the invention relates to the compounds of formula (I) for which S represents a monosaccharide or disaccharide, the hydroxyl function in position 3 of which is free.

Advantageously, the preferred monosaccharides are chosen from D-glucose, D-galactose, D-mannose, D-fucose, L-fucose, D-xylose, D-lyxose, D-glucuronic acid, D-galacturonic acid, D-iduronic acid, N-acetyl-D-glucosamine and N-acetyl-D-galactosamine; preferably D-glucose, D-xylose, D-galactose, D-fucose and L-fucose, and more preferentially D-glucose.

Another particular aspect of the invention relates to the compounds of formula (I) for which S represents a polysaccharide as defined above.

Advantageously, the preferred polysaccharides contain up to 6 sugar units and are chosen from D-maltose, D-lactose, D-cellobiose, D-maltotriose, a disaccharide combining a uronic acid chosen from D-iduronic acid, D-glucuronic acid and D-galacturonic acid with a hexosamine chosen from D-galactosamine, D-glucosamine, N-acetyl-D-galactosamine, N-acetyl-D-glucosamine, an oligosaccharide containing at least one xylose advantageously chosen from xylobiose, methyl-β-xylobioside, xylotriose, xylotetraose, xylopentaose and xylohexaose and preferentially xylobiose, which is composed of two xylose molecules linked via a 1-4 bond. As particularly preferred polysaccharides, mention may be made of D-maltose and D-lactose.

Among the preferred alkyl groups, mention may be made of methyl, ethyl, propyl, isopropyl, butyl, isobutyl, tert-butyl, sec-butyl, pentyl, n-hexyl, cyclopropyl, cyclopentyl, cyclohexyl and allyl groups, preferentially methyl, ethyl, isopropyl and tert-butyl groups, and more preferentially a methyl group.

The preferred aryl group is the phenyl group.

The preferred compounds of formulae (I) and (I') are those for which:
- X denotes a group chosen from =O, -OH, -NH₂ and -NHCH₃;
- Z denotes a group chosen from -NH₂, -CH₃, -OH and -OCH₃;
- Y denotes a group -OR₃ as defined above;
- m denotes an integer equal to 0, 1 or 2.

The compounds of formulae (I) and (I') that are particularly preferred are those for which:
- S denotes a saccharide residue chosen from glucose, mannose, xylose, fucose, galactose, maltose, lactose, rhamnose, lyxose, arabinose, N-acetylglucosamine and N-acetylgalactosamine; and preferably chosen from glucose, xylose, galactose and fucose; and preferentially glucose;
- X denotes a group chosen from =O, -OH, -NH₂ and -NHCH₃; and preferentially =O or -OH;
- Y denotes a group chosen from -OPO₃H₂, -OSO₃H, -OAC, -OH and -OCH₃; and more particularly -OH or -OCH₃;
- m = O.

The compounds of formulae (I) and (I') that are more particularly preferred are the following:

| |
|---|
| 2-deoxy-2-(2-hydroxy-2-(4-hydroxyphenyl)ethyl)-D(or L)-glucose (Example 2) |
| 2-deoxy-2-(2-hydroxy-2-(4-hydroxyphenyl)ethyl)-D(or L)-galactose |
| 2-deoxy-2-(2-hydroxy-2-(4-hydroxyphenyl)ethyl)-D(or L)-maltose |
| 2-deoxy-2-(2-hydroxy-2-(4-hydroxyphenyl)ethyl)-D(or L)-mannose |
| 2-deoxy-2-(2-hydroxy-2-(4-hydroxyphenyl)ethyl)-D(or L)-xylose |
| 2-deoxy-2-(2-hydroxy-2-(4-hydroxyphenyl)ethyl)-D(or L)-fucose |
| 2-deoxy-2-(2-hydroxy-2-(4-hydroxyphenyl)ethyl)-D(or L)-lactose |
| 2-deoxy-2-(2-hydroxy-2-(4-hydroxyphenyl)ethyl)-D(or L)-rhamnose |
| 2-deoxy-2-(2-hydroxy-2-(4-methoxyphenyl)ethyl)-D(or L)-glucose (Example 4) |
| 2-deoxy-2-(2-hydroxy-2-(4-methoxyphenyl)ethyl)-D(or L)-galactose |
| 2-deoxy-2-(2-hydroxy-2-(4-methoxyphenyl)ethyl)-D(or L)-maltose |
| 2-deoxy-2-(2-hydroxy-2-(4-methoxyphenyl)ethyl)-D(or L)-mannose |
| 2-deoxy-2-(2-hydroxy-2-(4-methoxyphenyl)ethyl)-D(or L)-xylose |
| 2-deoxy-2-(2-hydroxy-2-(4-methoxyphenyl)ethyl)-D(or L)-fucose |
| 2-deoxy-2-(2-hydroxy-2-(4-methoxyphenyl)ethyl)-D(or L)-lactose |
| 2-deoxy-2-(2-hydroxy-2-(4-methoxyphenyl)ethyl)-D(or L)-rhamnose |
| 2-deoxy-2- (2-oxo-2- (4-hydroxyphenyl) ethyl) -D (or L)-glucose (Example 1) |
| 2-deoxy-2-(2-oxo-2-(4-hydroxyphenyl)ethyl)-D(or L)-galactose |
| 2-deoxy-2-(2-oxo-2-(4-hydroxyphenyl)ethyl)-D(or L)-maltose |
| 2-deoxy-2-(2-oxo-2-(4-hydroxyphenyl)ethyl)-D(or L)-mannose |
| 2-deoxy-2-(2-oxo-2-(4-hydroxyphenyl)ethyl)-D(or L)-xylose |
| 2-deoxy-2-(2-oxo-2-(4-hydroxyphenyl)ethyl)-D(or L)-fucose |
| 2-deoxy-2-(2-oxo-2-(4-hydroxyphenyl)ethyl)-D(or L)-lactose |
| 2-deoxy-2-(2-oxo-2-(4-hydroxyphenyl)ethyl)-D(or L)-rhamnose |
| 2-deoxy-2-(2-oxo-2-(4-methoxyphenyl)ethyl)-D(or L)-glucose (Example 3) |
| 2-deoxy-2-(2₋oxo-2-(4-methoxyphenyl)ethyl)-D(or L)-galactose |
| 2-deoxy-2-(2-oxo-2-(4-methoxyphenyl)ethyl)-D(or L)-maltose |
| 2-deoxy-2-(2-oxo-2-(4-methoxyphenyl)ethyl)-D(or L)-mannose |
| 2-deoxy-2-(2-oxo-2-(4-methoxyphenyl)ethyl)-D(or L)-xylose |
| 2-deoxy-2-(2-oxo-2-(4-methoxyphenyl)ethyl)-D(or L)-fucose |
| 2-deoxy-2-(2-oxo-2-(4-methoxyphenyl)ethyl)-D(or L)-lactose |
| 2-deoxy-2-(2-oxo-2-(4-methoxyphenyl)ethyl)-D(or L)-rhamnose |
| 2-deoxy-2-(2-hydroxy-2-(3-methoxy-4-hydroxyphenyl)-ethyl)-D(or L)-glucose |
| 2-deoxy-2-(2-hydroxy-2-(3-methoxy-4-hydroxyphenyl)-ethyl)-D(or L)-galactose |
| 2-deoxy-2-(2-hydroxy-2-(3-methoxy-4-hydroxyphenyl)-ethyl)-D(or L)-maltose |
| 2-deoxy-2-(2-hydroxy-2-(3-methoxy-4-hydroxyphenyl)-ethyl)-D(or L)-mannose |
| 2-deoxy-2-(2-hydroxy-2-(3-methoxy-4-hydroxyphenyl)-ethyl)-D(or L)-xylose |
| 2-deoxy-2-(2-hydroxy-2-(3-methoxy-4-hydroxyphenyl)-ethyl)-D(or L)-fucose |
| 2-deoxy-2-(2-hydroxy-2-(3-methoxy-4-hydroxyphenyl)-ethyl)-D(or L)-lactose |
| 2-deoxy-2-(2-hydroxy-2-(3-methoxy-4-hydroxyphenyl)-ethyl)-D(or L)-rhamnose |
| 2-deoxy-2-(2-(4-hydroxyphenyl)ethyl)-D(or L)-glucose |
| 2-deoxy-2-(2-(4-hydroxyphenyl)ethyl)-D(or L)-galactose |
| 2-deoxy-2-(2-(4-hydroxyphenyl)ethyl)-D(or L)-maltose |
| 2-deoxy-2-(2-(4-hydroxyphenyl)ethyl)-D(or L)-mannose |
| 2-deoxy-2-(2-(4-hydroxyphenyl)ethyl)-D(or L)-xylose |
| 2-deoxy-2-(2-(4-hydroxyphenyl)ethyl)-D(or L)-fucose |
| 2-deoxy-2-(2-(4-hydroxyphenyl)ethyl)-D(or L)-lactose |
| 2-deoxy-2-(2-(4-hydroxyphenyl)ethyl)-D(or L)-rhamnose |
| 2-deoxy-2-(2-methylamino-2-(4-hydroxyphenyl)ethyl)-D(or L)-glucose |
| 2-deoxy-2-(2-methylamino-2-(4-hydroxyphenyl)ethyl)-D(or L)-galactose |
| 2-deoxy-2-(2-methylamino-2-(4-hydroxyphenyl)ethyl)-D(or L)-maltose |
| 2-deoxy-2-(2-methylamino-2-(4-hydroxyphenyl)ethyl)-D(or L)-mannose |
| 2-deoxy-2-(2-methylamino-2-(4-hydroxyphenyl)ethyl)-D(or L)-xylose |
| 2-deoxy-2-(2-methylamino-2-(4-hydroxyphenyl)ethyl)-D(or L)-fucose |
| 2-deoxy-2-(2-methylamino-2-(4-hydroxyphenyl)ethyl)-D(or L)-lactose |
| 2-deoxy-2-(2-methylamino-2-(4-hydroxyphenyl)ethyl)-D(or L)-rhamnose |
| 2-deoxy-2-(2-amino-2-(4-hydroxyphenyl)ethyl)-D(or L)-glucose |
| 2-deoxy-2-(2-amino-2-(4-hydroxyphenyl)ethyl)-D(or L)-galactose |
| 2-deoxy-2-(2-amino-2-(4-hydroxyphenyl)ethyl)-D(or L)-maltose |
| 2-deoxy-2-(2-amino-2-(4-hydroxyphenyl)ethyl)-D(or L)-mannose |
| 2-deoxy-2-(2-amino-2-(4-hydroxyphenyl)ethyl)-D(or L)-xylose |
| 2-deoxy-2-(2-amino-2-(4-hydroxyphenyl)ethyl)-D(or L)-fucose |
| 2-deoxy-2-(2-amino-2-(4-hydroxyphenyl)ethyl)-D(or L)-lactose |
| 2-deoxy-2-(2-amino-2-(4-hydroxyphenyl)ethyl)-D(or L)-rhamnose |
| 2-deoxy-2-(2-hydroxy-2-(4-hydroxyphenyl)ethyl)-D(or L)-lyxose |
| 2-deoxy-2-(2-hydroxy-2-(4-methoxyphenyl)ethyl)-D(or L)-lyxose |
| 2-deoxy-2-(2-oxo-2-(4-hydroxyphenyl)ethyl)-D(or L)-lyxose |
| 2-deoxy-2-(2-oxo-2-(4-methoxyphenyl)ethyl)-D(or L)-lyxose |
| 2-deoxy-2-(2-hydroxy-2-(3-methoxy-4-hydroxyphenyl)-ethyl)-D(or L)-lyxose |
| 2-deoxy-2-(2-(4-hydroxyphenyl)ethyl)-D(or L)-lyxose |
| 2-deoxy-2-(2-methylamino-2-(4-hydroxyphenyl)ethyl)-D(or L)-lyxose |
| 2-deoxy-2-(2-amino-2-(4-hydroxyphenyl)ethyl)-D(or L)-lyxose |
| 2-deoxy-2-(2-hydroxy-2-(4-hydroxyphenyl)ethyl)-D(or L)-arabinose |
| 2-deoxy-2-(2-hydroxy-2-(4-methoxyphenyl)ethyl)-D(or L)-arabinose |
| 2-deoxy-2-(2-oxo-2-(4-hydroxyphenyl)ethyl)-D(or L)-arabinose |
| 2-deoxy-2-(2-oxo-2-(4-methoxyphenyl)ethyl)-D(or L)-arabinose |
| 2-deoxy-2-(2-hydroxy-2-(3-methoxy-4-hydroxyphenyl)-ethyl)-D(or L)-arabinose |
| 2-deoxy-2-(2-(4-hydroxyphenyl)ethyl)-D(or L)-arabinose |
| 2-deoxy-2-(2-methylamino-2-(4-hydroxyphenyl)ethyl)-D(or L)-arabinose |
| 2-deoxy-2-(2-amino-2-(4-hydroxyphenyl)ethyl)-D(or L)-arabinose |
| 2-deoxy-2-(2-hydroxy-2-(4-hydroxyphenyl)ethyl)-D(or L)-N-acetylglucosamine |
| 2-deoxy-2-(2-hydroxy-2-(4-methoxyphenyl)ethyl)-D(or L)-N-acetylglucosamine |
| 2-deoxy-2-(2-oxo-2-(4-hydroxyphenyl)ethyl)-D(or L)-N-acetylglucosamine |
| 2-deoxy-2-(2-oxo-2-(4-methoxyphenyl)ethyl)-D(or L)-N-acetylglucosamine |
| 2-deoxy-2-(2-hydroxy-2-(3-methoxy-4-hydroxyphenyl)-ethyl)-D(or L)-N-acetylglucosamine |
| 2-deoxy-2-(2-(4-hydroxyphenyl)ethyl)-D(or L)-N-acetylglucosamine |
| 2-deoxy-2-(2-methylamino-2-(4-hydroxyphenyl)ethyl)-D(or L)-N-acetylglucosamine |
| 2-deoxy-2-(2-amino-2-(4-hydroxyphenyl)ethyl)-D(or L)-N-acetylglucosamine |
| 2-deoxy-2-(2-hydroxy-2-(4-hydroxyphenyl)ethyl)-D(or L)-N-acetylgalactosamine |
| 2-deoxy-2-(2-hydroxy-2-(4-methoxyphenyl)ethyl)-D(or L)-N-acetylgalactosamine |
| 2-deoxy-2-(2-oxo-2-(4-hydroxyphenyl)ethyl)-D(or L)-N-acetylgalactosamine |
| 2.-deoxy-2-(2-oxo-2-(4-methoxyphenyl)ethyl)-D(or L)-N-acetylgalactosamine |
| 2-deoxy-2-(2-hydroxy-2-(3-methoxy-4-hydroxyphenyl)-ethyl)-D(or L)-N-acetylgalactosamine |
| 2-deoxy-2-(2-(4-hydroxyphenyl)ethyl)-D(or L)-N-acetylgalactosamine |
| 2-deoxy-2-(2-met.hylamino-2-(4-hydroxyphenyl)ethyl)-D(or L)-N-acetylgalactosamine |
| 2-deoxy-2-(2-amino-2-(4-hydroxyphenyl)ethyl)-D(or L)-N-acetylgalactosamine |

The compound (I) and (I') that are particularly preferred are chosen from:
2-deoxy-2-(2-hydroxy-2-(4-hydroxyphenyl)ethyl)-D(or L)-glucose
2-deoxy-2-(2-hydroxy-2-(4-methoxyphenyl)ethyl)-D(or L)-glucose
2-deoxy-2-(2-oxo-2-(4-methoxyphenyl)ethyl)-D(or L)-glucose
2-deoxy-2-(2-oxo-2-(4-hydroxyphenyl)ethyl)-D(or L)-glucose.

According to a first particular synthetic mode (Scheme I), the compounds of formula (I) for which C-X = C=O may be prepared by reacting a glycoside (II) with a diketo compound (III) in the presence of a base, at a temperature especially ranging from 70°C to 100°C. The reaction may be performed in water or in a water/alcohol medium (such as ethanol, isopropanol or methanol). After cooling, the organic solvents possibly present are evaporated off and the residual aqueous phase is then diluted with water, then passed through an acidic resin and subsequently concentrated to isolate the compound of formula (Ia) obtained.

Bases that may be used include a mineral base such as sodium hydroxide, potassium hydroxide, sodium bicarbonate, potassium bicarbonate, sodium carbonate or potassium carbonate.

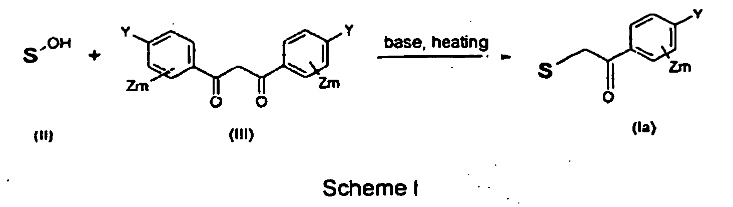

The diketo compound (III) may be prepared according to the synthetic scheme Ia, by reacting an ester (IIIa) (the alkyl group especially being a C₁-C₄ alkyl and in particular methyl or ethyl) and a ketone (IIIb) deprotonated beforehand with a strong base (for instance sodium hydroxide or lithium diisopropylamide), especially at a temperature of between 20°C and 100°C. The reaction may be performed in an ether solvent (for instance dioxane, tetrahydrofuran or diisopropyl ether) or in a polar solvent (for instance dimethylformamide).

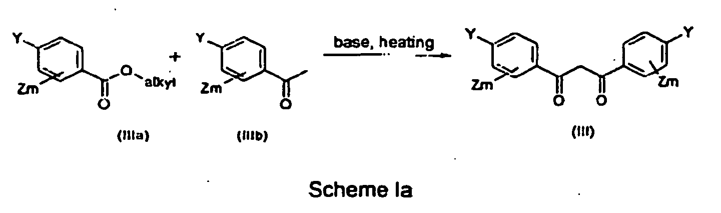

According to a second particular synthetic mode, the compound of formula (I) for which C-X = C-OH may be prepared according to Scheme (II) via reduction of the keto compound (Ia) obtained according to the preparation mode described above (Scheme (I)) in the presence of a metal hydride such as sodium borohydride, lithium aluminium hydride or sodium bis(2-methoxyethoxy)-aluminium hydride (sold under the name Red-Al by the company Aldrich), especially in alcohol (such as ethanol, isopropanol or methanol) or an ether solvent (for instance dioxane, tetrahydrofuran or diisopropyl ether) at room temperature, for 1 to 20 hours. After reaction, the excess mental hydride is destroyed by adding acetone. After purification, compound (Ib) is obtained in the form of a mixture of diastereoisomers.

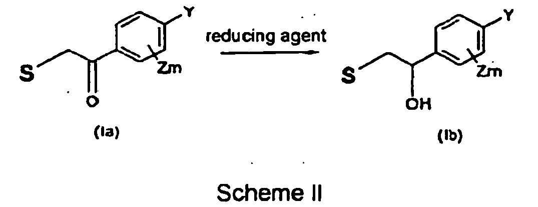

According to a third particular synthetic mode (Scheme III), the compounds of formula (I) for which C-X = C-NR₁R₂ may be prepared by reacting the keto compound (Ia) obtained according to the preparation mode described above (Scheme (I)) with an amine HNR₁R₂, especially in alcohol (such as ethanol or isopropanol) at a temperature of between 20°C and 80°C, optionally in the presence of a drying agent such as magnesium sulfate, sodium sulfate or molecular sieves. The reaction is advantageously performed for 0.5 to 10 hours. A metal hydride (as indicated above) is then added portionwise, especially while allowing the reaction to proceed at room temperature for 1 to 20 hours. After reaction, the excess metal hydride is destroyed by adding acetone. After purification, compound (Ic) is obtained in the form of a mixture of diastereoisomers.

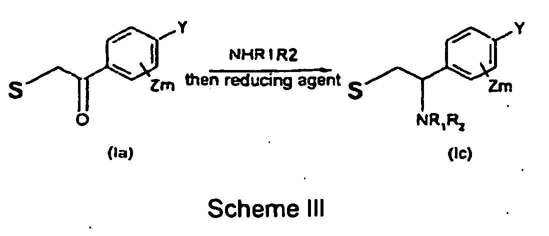

According to a fourth particular synthetic mode (Scheme IV), the compounds of formula (II) for which C-X = CH₂ may be prepared by reduction with activated zinc of the keto compound (Ia) obtained according to the preparation mode described above (Scheme (I)) and dissolved in acetic acid saturated with gaseous hydrogen chloride. The reaction is preferably first performed at about 0°C and is then allowed to warm to room temperature and is left to react for about 10 hours. After purification, compound (Id) is obtained.

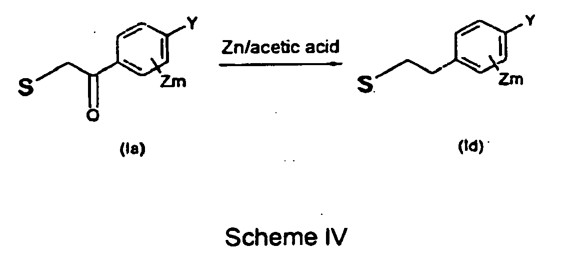

According to a fifth particular synthetic mode (Scheme V), the compounds of formula (I) for which C-X = C-OR₁ may be prepared according to the following sequence:
Step 1: Protection of the OH functions of the keto compound (Ia) obtained according to the preparation mode described above (Scheme (I)), in acetic anhydride in the presence of a catalytic amount of zinc chloride.
Step 2: Reduction according to a synthetic mode identical to that described for Scheme (II).
Step 3: Reaction of the intermediate compound with a strong base (for instance sodium hydride) and then with a compound R₁-X (X being a labile group such as halide, mesylate or tosylate), at a temperature of between 20°C and 100°C. The reaction may be performed in an ether solvent (for instance dioxane, tetrahydrofuran or diisopropyl ether) or in an polar solvent (for instance dimethylformamide).
Step 4: Deprotection of the OAc functions with a mineral base (sodium hydroxide or lithium hydroxide) in a water/alcohol mixture (the alcohol possibly being methanol or ethanol) at a temperature of from 20°C to 100°C, to obtain the compound of formula (Ie).

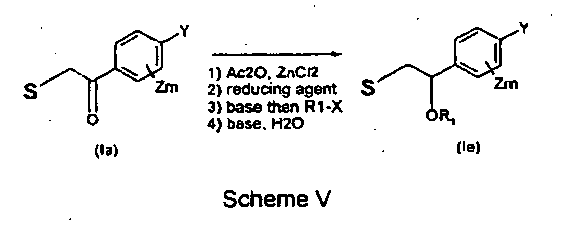

Another subject of the invention relates to a composition comprising, in a physiologically acceptable medium, at least one C-glycoside derivative corresponding to formula (I) as defined above. In particular, the composition is suitable for topical application to the skin. The physiologically acceptable medium will preferentially be a cosmetically or dermatologically acceptable medium, i.e. a medium with no unpleasant odour, colour or appearance, and which does not cause any stinging, tautness or redness that is unacceptable to the user.

The term "physiologically acceptable medium" means a medium that is compatible with human keratin materials, for instance the skin, mucous membranes, the nails, the scalp and/or the hair.

The composition according to the invention may be intended for cosmetic or pharmaceutical application, particularly dermatological application. The composition according to the invention is preferably intended for cosmetic application.

The amount of compounds of formula (I) that may be used in the context of the invention obviously depends on the desired effect.

By way of example, this amount may range, for example, from 0.001% to 10% by weight, preferably from 0.01% to 5% by weight and especially from 0.1°s to 2% by weight relative to the total weight of the composition.

The composition may then comprise any constituent usually used in the intended application.

Mention may be made especially of water, solvents, oils of mineral, animal and/or plant origin, waxes, pigments, fillers, surfactants, cosmetic or dermatological active agents, UV-screening agents, polymers, gelling agents and preserving agents.

Needless to say, a person skilled in the art will take care to select this or these optional additional compound(s), and/or the amount thereof, such that the advantageous properties of the compounds according to the invention are not, or are not substantially, adversely affected by the envisaged addition.

The composition according to the invention may be in any pharmaceutical form normally used in cosmetics and dermatology, in particular in the form of an optionally gelled aqueous or aqueous-alcoholic solution, an optionally two-phase lotion-type dispersion, an oil-in-water or water-in-oil or multiple emulsion, an aqueous gel, a dispersion of oil in an aqueous phase with the aid of spherules, these spherules possibly being polymeric nanoparticles such as nanospheres and nanocapsules or better still lipid vesicles of ionic and/or non-ionic type.

When the composition of the invention is an emulsion, the proportion of the fatty phase can range from 5 to 80% by weight, and preferably from 5 to 50% by weight, relative to the total weight of the composition. The oils, the emulsifiers and the optional co-emulsifiers used in the composition in emulsion form are chosen from those used conventionally in the field considered. The emulsifier and the co-emulsifier are present in the composition in a proportion ranging from 0.3 to 30% by weight, and preferably from 0.5 to 20% by weight, relative to the total weight of the composition.

This composition may be relatively fluid and have the appearance of a white or coloured cream, an ointment, a milk, a lotion, a serum, a paste or a foam. It may optionally be applied to the skin in aerosol form. It may also be in solid form and, for example, in the form of a stick. It can be used as a care product and/or as a make-up product.

This composition may constitute a cleansing, protective, treatment or care cream for the face, the hands, the feet, the major anatomical folds or the body (for example day creams, night creams, makeup-removing creams, foundation creams or antisun creams); a fluid foundation, a makeup-removing milk, a protective or care body milk or an antisun milk; or a skincare lotion, gel or mousse, such as a cleansing lotion.

In one advantageous aspect of the invention, the compositions used may also comprise at least one desquamating agent, and/or at least one calmative and/or at least one organic photoprotective agent and/or at least one mineral photoprotective agent.

The term "desquamating agent" means any compound capable of acting:
- either directly on the desquamation by promoting exfoliation, such as β-hydroxyl acids, in particular salicylic acid and its derivatives (including 5-n-octanoylsalicylic acid); α-hydroxy acids, such as glycolic acid, citric acid, lactic acid, tartaric acid, malic acid or mandelic acid; urea; gentisic acid; oligofucoses; cinnamic acid; extract of Saphora japonica; resveratrol;
- or on the enzymes involved in the desquamation or degradation of corneodesmosomes, glycosidases, stratum corneum chymotryptic enzyme (SCCE), or even other proteases (trypsin, chymotrypsin-like). Mention may be made of agents for chelating mineral salts: EDTA; N-acyl-N,N',N'-ethylenediaminetriacetic acid; amino-sulfonic compounds and in particular (N-2-hydroxyethylpiperazine-N-2-ethane)sulfonic acid (HEPES); derivatives of 2-oxothiazolidine-4-carboxylic acid (procysteine); derivatives of α-amino acids of the type such as glycine (as described in EP-0 852 949, and sodium methylglycinediacetate sold by BASF under the trade name Trilon M) ; honey; sugar derivatives such as O-octanoyl-6-D-maltose and N-acetylglucosamine.

As calmatives that may be used in the composition according to the invention, mention may be made of: pentacyclic triterpenes and extracts of plants (e.g.: Glycyrrhiza glabra) containing them, for instance β-glycyrrhetinic acid and salts and/or derivatives thereof (glycyrrhetinic acid monoglucoronide, stearyl glycyrrhetinate or 3-stearoyloxyglycyrrhetic acid), ursolic acid and its salts, oleanolic acid and its salts, betulinic acid and its salts, an extract of Paeonia suffruticosa and/or lactiflora, salicylic acid salts and in particular zinc salicylate, the phycosaccharides from the company Codif, an extract of Laminaria saccharina, canola oil, bisabolol and camomile extracts, allantoin, Sepivital EPC (phosphoric diester of vitamins E and C) from SEPPIC, omega-3 unsaturated oils such as musk rose oil, blackcurrant oil, ecchium oil, fish oil, plankton extracts, capryloylglycine, Seppicalm VG (sodium palmitoylproline and Nymphea alba) from SEPPIC, an extract of Pygeum, an extract of Boswellia serrata, an extract of Centipeda cunnighami, an extract of Helianthus annuus, an extract of Linum usitatissimum, tocotrienols, extracts of Cola nitida, piperonal, an extract of clove, an extract of Epilobium angustifolium, Aloe vera, an extract of Bacopa moniera, phytosterols, cortisone, hydrocortisone, indomethacin and betamethasone.

The organic photoprotective agents are chosen especially from anthranilates; cinnamic derivatives; dibenzoylmethane derivatives; salicylic derivatives, camphor derivatives; triazine derivatives such as those described in patent applications US 4 367 390, EP 863 145, EP 517 104, EP 570 838, EP 796 851, EP 775 698, EP 878 469, EP 933 376, EP 507 691, EP 507 692, EP 790 243 and EP 944 624; benzophenone derivatives; β,β-diphenylacrylate derivatives; benzotriazole derivatives; benzalmalonate derivatives; benzimidazole derivatives; imidazolines; bis-benzazolyl derivatives as described in patents EP 669 323 and US 2 463 264; p-aminobenzoic acid (PABA) derivatives; methylenebis(hydroxyphenylbenzotriazole) derivatives as described in patent applications US 5 237 071, US 5 166 355, GB 2 303 549, DE 197 26 184 and EP 893 119; and screening polymers and screening silicones such as those described especially in patent application WO 93/04665; dimers derived from α-alkyl-styrene, such as those described in patent application DE 198 55 649.

The mineral photoprotective agents are chosen from pigments or nanopigments (mean size of the primary particles: generally between 5 nm and 100 nm and preferably between 10 nm and 50 nm) of coated or uncoated metal oxides, for instance nanopigments of titanium oxide (amorphous or crystallized in rutile and/or anatase form), of iron oxide, of zinc oxide, of zirconium oxide or of cerium oxide, which are all UV photoprotective agents that are well known per se. Standard coating agents are moreover alumina and/or aluminium stearate. Such coated or uncoated metal oxide nanopigments are described in particular in patent applications EP 518 772 and EP 518 773.

The photoprotective agents are generally present in the composition according to the invention in proportions ranging from 0.1% to 20% by weight relative to the total weight of the composition, and preferably ranging from 0.2% to 15% by weight relative to the total weight of the composition.

The examples that follow illustrate the invention without limiting its scope. Depending on the case, the compounds are cited as the chemical names or as the CTFA names (International Cosmetic Ingredient Dictionary and Handbook).

### Example 1:

### Synthesis of 2-deoxy-2-(2-oxo-2-(4-hydroxyphenyl)ethyl)-D(or L)-glucose

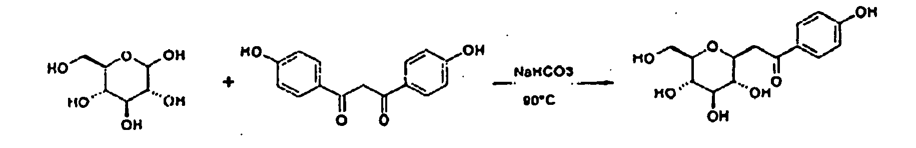

D-Glucose (60 mg, 0.33 mmol), water (0.25 mL) and sodium bicarbonate (NaHCO₃) were introduced into a round-bottomed flask, and 1,3-bis(4-hydroxyphenyl)-1,3-propanedione (127 mg, 0.5 mmol) dissolved in ethanol (0.75 mL) was then added. The mixture was heated at 90°C for 20 hours. The medium was then cooled and the ethanol was evaporated off. The aqueous phase was diluted, extracted with dichloromethane, passed through Dowex® 50WX2-200 acid resin from Lancaster, and then concentrated. The residue (120 mg) obtained is a paste and is identified as being 2-deoxy-2-(2-oxo-2-(4-hydroxyphenyl) ethyl)-D(or L) -glucose.

The mass spectrometry is in accordance with the structure of the expected product.

### Example 2:

### Synthesis of 2-deoxy-2-(2-hydroxy-2-(4-hydroxyphenyl)-ethyl)-D(or L)-glucose

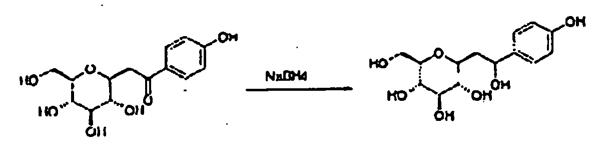

The crude product obtained in Example 1 (120 mg) was introduced into a round-bottomed flask and dissolved in methanol (5 mL). NaBH₄ (60 mg) was added portionwise and the mixture was then stirred at room temperature for 20 hours. Acetone (20 mL) was added to the medium. The medium was stirred for 1 hour and then concentrated. The residue was purified on a column of silica gel (for example silica gel 60 (0.040-0:063 mm from Merck) (eluent: 4/1 CH₂Cl₂/MeOH) to give 2-deoxy-2-(2-hydroxy-2-(4-hydroxyphenyl)ethyl)-D(or L)-glucose (80 mg) in the form of a paste containing 2 major diastereoisomers.

The analyses performed (¹H NMR; gas chromatography; mass spectrum) are in accordance with the expected structure of the product.

### Example 3:

### Synthesis of 2-deoxy-2-(2-oxo-2-(4-methoxyphenyl)ethyl)-D(or L)-glucose

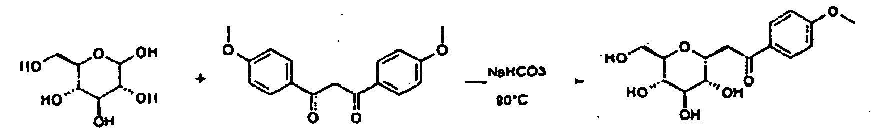

D-Glucose (2 g, 11.1 mmol), water (8 mL) and sodium bicarbonate (1.4 g, 16.7 mmol) were introduced into a round-bottomed flask, and 1,3-bis(9-methoxyphenyl)-1,3-propanedione (CAS 18362-51-1) (4.7 g, 16.7 mmol) dissolved in ethanol (25 mL) was then added. The mixture was heated at 90°C for 20 hours. The medium was then cooled and the ethanol was evaporated off. The aqueous phase was diluted, extracted with dichloromethane, passed through a Dowex® 50WX2-200 resin from Lancaster, and then concentrated. 1.8 g of 2-deoxy-2-(2-oxo-2-(4-methoxyphenyl)ethyl)-D(or L)-glucose were obtained.

The analyses performed (¹H NMR, mass spectrum) are in accordance with the expected structure.

### Example 4:

### Synthesis of 2-deoxy-2-(2-hydroxy-2-(4-methoxyphenyl)-ethyl)-D(or L)-glucose

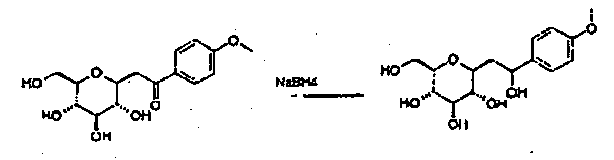

The product obtained in Example 3 (800 mg, 2.56 mmol) was introduced into a round-bottomed flask and dissolved in methanol (15 mL). NaBH₄ (114 mg, 3 mmol) was added portionwise and the mixture was then stirred at room temperature for 3 hours. Acetone was added to the medium, which was stirred for 1 hour and then concentrated. The residue obtained was purified on a column of silica gel (for example silica gel 60 (0.040-0.063 mm from Merck) (eluent: 4/1 CH₂Cl₂/MeOH), to give 2-deoxy-2-(2-hydroxy-2-(4-methoxyphenyl)ethyl)-D(or L)-glucose (798 mg) in the form of an oil containing a mixture of several diastereoisomers.

The analyses performed (¹H NMR, mass spectrum) are in accordance with the expected structure.

### Example 5: Demonstration of the activity on melanogenesis

A biological test demonstrate the depigmenting activity of the compound of Example 2.

The melanogenesis-modulating effect of the compound of Example 2 was measured according to the method described in patent FR-A-2 734 825, and also in the article by R. Schmidt, P. Krien and M. Régnier, Anal. Biochem., 235(2), 113-18, (1996). This test is performed on a co-culture of keratinocytes and melanocytes.

The following were determined for the test compound:
- the cytotoxicity, by estimating the incorporation of leucine,
- the inhibitory activity on melanin synthesis, by estimating the ratio of the incorporation of thiouracil to the incorporation of leucine, relative to 100% of the control (the control corresponds to the test performed without test compound). The IC₅₀ values (the concentration for which a 50% inhibition of melanogenesis is observed) were also determined.

The test was also performed with arbutin, which is a known depigmenting compound.

The results are collated in the following table:

| | Cytotoxicity | IC₅₀ (µM) |
|---|---|---|
| Compound of Ex. 2 | No | 100 |
| Arbutin | No | 100 |

The compound of Example 2 thus proves to be efficient in inhibiting melanogenesis; it is moreover more efficient than arbutin.

### Example 6

A facial care bleaching cream of oil-in-water emulsion type is prepared, comprising (% by weight):
- compound of Example 1 0.005%
- glyceryl stearate 2%
- Polysorbate 60 (Tween 60 from ICI) 1%
- stearic acid 1.4%
- triethanolamine 0.7%
- carbomer 0.4%
- liquid fraction of shea butter 12%
- perhydrosqualene 12%
- antioxidant 0.05%
- fragrance, preserving agent qs
- water qs 100% A similar composition is prepared with the compound of Example 3.

### Example 7

A skin depigmenting gel is prepared, comprising (% by weight):
- compound of Example 2 2%
- hydroxypropylcellulose (Klucel H from Hercules) 1%
- antioxidant 0.05%
- isopropanol 40%
- fragrance, preserving agent qs
- water qs 100%

A similar composition is prepared with the compound of Example 4.

## Claims

1. Composition comprising, in a physiologically acceptable medium, at least one compound of formula (I): 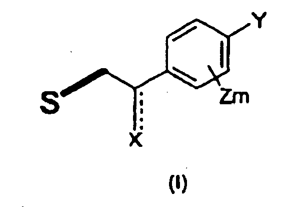 in which:
- S represents a monosaccharide or a polysaccharide containing up to 20 sugar units, in pyranose and/or furanose form and of L and/or D series, the said monosaccharide or polysaccharide containing at least one hydroxyl function that is necessarily free and/or optionally one or more optionally protected amine functions;
- the bond S-CH₂ being a C-glycoside bond;
- X represents a hydrogen atom or a group chosen from -OR₁ and -NR₁R₂ or an =0 group;
- Y represents a group chosen from -OR₃, -NR₁R₂ and -OSi (R₄) ₃;
- Z represents a fluorine atom or a group chosen from a linear C₁-C₆ alkyl group, a cyclic or branched C₃-C₆ alkyl group, -OR₁, -NR₁R₂, -COR₁, -CO₂R₁, -CONR₁R₂ and -CR₃;
- m is an integer ranging from 0 to 4;
with
- R₁ and R₂ denoting, independently of each other, a hydrogen atom or a linear C₁-C₆ alkyl group, a cyclic or branched C₃-C₆ alkyl groupe, aralkyl or phenyl;
- R₃ denoting a hydrogen atom or a linear C₁-C₆ alkyl group, a cyclic or branched C₃-C₆ alkyl group, aralkyl or phenyl, or a group -SO₃H -PO₃H₂ or -COR₁;
- R₄ denoting a linear C₁-C₆ alkyl group, a cyclic or branched C₃-C₆ alkyl group or phenyl;
and also the cosmetically acceptable salts thereof, solvates thereof such as hydrates;
and a constituent chosen among water, oils of mineral, animal and/or plant origin, waxes, pigments, fillers, surfactants, UV-screening agents, polymers, gelling agents.

2. Composition according to the preceding claim, **characterized in that** S represents a monosaccharide or disaccharide, the hydroxyl function in position 3 of which is free.

3. Composition according to either of the preceding claims, **characterized in that** S represents a monosaccharide chosen from D-glucose, D-galactose, D-mannose, D-fucose, L-fucose, D-xylose, D-lyxose, D-glucuronic acid, D-galacturonic acid, D-iduronic acid, N-acetyl-D-glucosamine and N-acetyl-D-galactosamine.

4. Composition according to any one of the preceding claims, **characterized in that** S represents D-glucose.

5. Composition according to Claim 1 or 2, **characterized in that** S represents a polysaccharide containing up to 6 sugar units.

6. Composition according to any one of Claims 1, 2 and 5, **characterized in that** S represents a polysaccharide chosen from D-maltose, D-lactose, D-cellobiose, D-maltotriose, a disaccharide combining a uronic acid chosen from D-iduronic acid, D-glucuronic acid and D-galacturonic acid with a hexosamine chosen from D-galactosamine, D-glucosamine, N-acetyl-D-galactosamine, N-acetyl-D-glucosamine, an oligosaccharide containing at least one xylose.

7. Composition according to any one of Claims 1, 2, 5 and 6, **characterized in that** S represents a polysaccharide chosen from D-maltose and D-lactose.

8. Composition according to any one of the preceding claims, **characterized in that**:
- X denotes a group chosen from =O, -OH, -NH₂ and -NHCH₃;
- Z denotes a group chosen from -NH₂, -CH₃, -OH and -OCH₃;
- Y denotes a group -OR₃ as defined in Claim 1;
- m denotes an integer equal to 0, 1 or 2.

9. Composition according to any one of the preceding claims, **characterized in that**:
- S denotes a saccharide residue chosen from glucose, mannose, xylose, fucose, galactose, maltose, lactose, rhamnose, lyxose, arabinose, N-acetylglucosamine and N-acetylgalactosamine;
- X denotes a group chosen from =O, -OH, -NH₂ and -NHCH₃;
- Y denotes a group chosen from -OPO₃H₂, -OSO₃H, -OAc, -OH and -OCH₃;
- m = 0.

10. Composition according to any one of the preceding claims, **characterized in that** the compound of formula (I) is chosen from:
2-deoxy-2-(2-hydroxy-2-(9-hydroxyphenyl)ethyl)-D(or L)-glucose
2-deoxy-2-(2-hydroxy-2-(4-hydroxyphenyl)ethyl)-D(or L)-galactose
2-deoxy-2-(2-hydroxy-2-(4-hydroxyphenyl)ethyl)-D(or L)-maltose
2-deoxy-2-(2-hydroxy-2-(4-hydroxyphenyl)ethyl)-D(or L)-mannose
2-deoxy-2-(2-hydroxy-2-(4-hydroxyphenyl)ethyl)-D(or L)-xylose
2-deoxy-2-(2-hydroxy-2-(4-hydroxyphenyl)ethyl)-D(or L)-fucose
2-deoxy-2-(2-hydroxy-2-(4-hydroxyphenyl)ethyl)-D(or L)-lactose
2-deoxy-2-(2-hydroxy-2-(4-hydroxyphenyl)ethyl)-D(or L)-rhamnose
2-deoxy-2-(2-hydroxy-2-(4-methoxyphenyl)ethyl)-D(or L)-glucose
2-deoxy-2-(2-hydroxy-2-(4-methoxyphenyl)ethyl)-D(or L)-galactose
2-deoxy-2-(2-hydroxy-2-(4-methoxyphenyl)ethyl)-D(or L)-maltose
2-deoxy-2-(2-hydroxy-2-(4-methoxyphenyl)ethyl)-D(or L)-mannose
2-deoxy-2-(2-hydroxy-2-(4-methoxyphenyl)ethyl)-D(or L)-xylose
2-deoxy-2-(2-hydroxy-2-(4-methoxyphenyl)ethyl)-D(or L)-fucose
2-deoxy-2-(2-hydroxy-2-(4-methoxyphenyl)ethyl)-D(or L)-lactose
2-deoxy-2-(2-hydroxy-2-(4-methoxyphenyl)ethyl)-D(or L)-rhamnose
2-deoxy-2-(2-oxo-2-(4-hydroxyphenyl)ethyl)-D(or L)-glucose
2-deoxy-2-(2-oxo-2-(4-hydroxyphenyl)ethyl)-D(or L)-galactose
2-deoxy-2-(2-oxo-2-(4-hydroxyphenyl)ethyl)-D(or L)-maltose
2-deoxy-2-(2-oxo-2-(4-hydroxyphenyl)ethyl)-D(or L)-mannose
2-deoxy-2-(2-oxo-2-(4-hydroxyphenyl)ethyl)-D(or L)-xylose
2-deoxy-2-(2-oxo-2-(4-hydroxyphenyl)ethyl)-D(or L)-fucose
2-deoxy-2-(2-oxo-2-(4-hydroxyphenyl)ethyl)-D(or L)-lactose
2-deoxy-2-(2-oxo-2-(4-hydroxyphenyl)ethyl)-D(or L)-rhamnose
2-deoxy-2-(2-oxo-2-(4-methoxyphenyl)ethyl)-D(or L)-glucose
2-deoxy-2-(2-oxo-2-(4-methoxyphenyl)ethyl)-D(or L)-galactose
2-deoxy-2-(2-oxo-2-(4-methoxyphenyl)ethyl)-D(or L)-maltose
2-deoxy-2-(2-oxo-2-(4-methoxyphenyl)ethyl)-D(or L)-mannose
2-deoxy-2-(2-oxo-2-(4-methoxyphenyl)ethyl)-D(or L)-xylose
2-deoxy-2-(2-oxo-2-(4-methoxyphenyl)ethyl)-D(or L)-fucose
2-deoxy-2-(2-oxo-2-(4-methoxyphenyl)ethyl)-D(or L)-lactose
2-deoxy-2-(2-oxo-2-(4-methoxyphenyl)ethyl)-D(or L)-rhamnose
2-deoxy-2-(2-hydroxy-2-(3-methoxy-4-hydroxyphenyl)-ethyl)-D(or L)-glucose
2-deoxy-2-(2-hydroxy-2-(3-methoxy-4-hydroxyphenyl)-ethyl)-D(or L)-galactose
2-deoxy-2-(2-hydroxy-2-(3-methoxy-4-hydroxyphenyl)-ethyl)-D(or L)-maltose
2-deoxy-2-(2-hydroxy-2-(3-methoxy-4-hydroxyphenyl)-ethyl)-D(or L)-mannose
2-deoxy-2-(2-hydroxy-2-(3-methoxy-4-hydroxyphenyl)-ethyl)-D(or L)-xylose
2-deoxy-2-(2-hydroxy-2-(3-methoxy-4-hydroxyphenyl)-ethyl)-D(or L)-fucose
2-deoxy-2-(2-hydroxy-2-(3-methoxy-4-hydroxyphenyl)-ethyl)-D(or L)-lactose
2-deoxy-2-(2-hydroxy-2-(3-methoxy-4-hydroxyphenyl)-ethyl)-D(or L)-rhamnose
2-deoxy-2-(2-(4-hydroxyphenyl)ethyl)-D(or L)-glucose
2-deoxy-2-(2-(4-hydroxyphenyl)ethyl)-D(or L)-galactose
2-deoxy-2-(2-(4-hydroxyphenyl)ethyl)-D(or L)-maltose
2-deoxy-2-(2-(4-hydroxyphenyl)ethyl)-D(or L)-mannose
2-deoxy-2-(2-(4-hydroxyphenyl)ethyl)-D(or L)-xylose
2-deoxy-2-(2-(4-hydroxyphenyl)ethyl)-D(or L)-fucose
2-deoxy-2-(2-(4-hydroxyphenyl)ethyl)-D(or L)-lactose
2-deoxy-2-(2-(4-hydroxyphenyl)ethyl)-D(or L)-rhamnose
2-deoxy-2-(2-methylamino-2-(4-hydroxyphenyl)ethyl)-D(or L)-glucose
2-deoxy-2-(2-methylamino-2-(4-hydroxyphenyl)ethyl)-D(or L)-galactose
2-deoxy-2-(2-methylamino-2-(4-hydroxyphenyl)ethyl)-D(or L)-maltose
2-deoxy-2-(2-methylamino-2-(4-hydroxyphenyl)ethyl)-D(or L)-mannose
2-deoxy-2-(2-methylamino-2-(4-hydroxyphenyl)ethyl)-D(or L)-xylose
2-deoxy-2-(2-methylamino-2-(4-hydroxyphenyl)ethyl)-D(or L)-fucose
2-deoxy-2-(2-methylamino-2-(4-hydroxyphenyl)ethyl)-D(or L)-lactose
2-deoxy-2-(2-methylamino-2-(4-hydroxyphenyl)ethyl)-D(or L)-rhamnose
2-deoxy-2-(2-amino-2-(4-hydroxyphenyl)ethyl)-D(or L)-glucose
2-deoxy-2-(2-amino-2-(4-hydroxyphenyl)ethyl)-D(or L)-galactose
2-deoxy-2-(2-amino-2-(4-hydroxyphenyl)ethyl)-D(or L)-maltose
2-deoxy-2-(2-amino-2-(4-hydroxyphenyl)ethyl)-D(or L)-mannose
2-deoxy-2-(2-amino-2-(4-hydroxyphenyl)ethyl)-D(or L)-xylose
2-deoxy-2-(2-amino-2-(4-hydroxyphenyl)ethyl)-D(or L)-fucose
2-deoxy-2-(2-amino-2-(4-hydroxyphenyl)ethyl)-D(or L)-lactose
2-deoxy-2-(2-amino-2-(4-hydroxyphenyl)ethyl)-D(or L)-rhamnose
2-deoxy-2-(2-hydroxy-2-(4-hydroxyphenyl)ethyl)-D(or L)-lyxose
2-deoxy-2-(2-hydroxy-2-(4-methoxyphenyl)ethyl)-D(or L)-lyxose
2-deoxy-2-(2-oxo-2-(4-hydroxyphenyl)ethyl)-D(or L)-lyxose
2-deoxy-2-(2-oxo-2-(4-methoxyphenyl)ethyl)-D(or L)-lyxose
2-deoxy-2-(2-hydroxy-2-(3-methoxy-4-hydroxyphenyl)-ethyl)-D(or L)-lyxose
2-deoxy-2-(2-(4-hydroxyphenyl)ethyl)-D(or L)-lyxose
2-deoxy-2-(2-methylamino-2-(4-hydroxyphenyl)ethyl)-D(or L)-lyxose
2-deoxy-2-(2-amino-2-(4-hydroxyphenyl)ethyl)-D(or L)-lyxose
2-deoxy-2-(2-hydroxy-2-(4-hydroxyphenyl)ethyl)-D(or L)-arabinose
2-deoxy-2-(2-hydroxy-2-(4-methoxyphenyl)ethyl)-D(or L)-arabinose
2-deoxy-2-(2-oxo-2-(4-hydroxyphenyl)ethyl)-D(or L)-arabinose
2-deoxy-2-(2-oxo-2-(4-methoxyphenyl)ethyl)-D(or L)-arabinose
2-deoxy-2-(2-hydroxy-2-(3-methoxy-4-hydroxyphenyl)-ethyl)-D(or L)-arabinose
2-deoxy-2-(2-(4-hydroxyphenyl)ethyl)-D(or L)-arabinose
2-deoxy-2-(2-methylamino-2-(4-hydroxyphenyl)ethyl)-D(or L)-arabinose
2-deoxy-2-(2-amino-2-(4-hydroxyphenyl)ethyl)-D(or L)-arabinose
2-deoxy-2-(2-hydroxy-2-(4-hydroxyphenyl)ethyl)-D(or L)-N-acetylglucosamine
2-deoxy-2-(2-hydroxy-2-(4-methoxyphenyl) ethyl)-D(or L)-N-acetylglucosamine
2-deoxy-2-(2-oxo-2-(4-hydroxyphenyl)ethyl)-D(or L)-N-acetylglucosamine
2-deoxy-2-(2-oxo-2-(4-methoxyphenyl)ethyl)-D(or L)-N-acetylglucosamine
2-deoxy-2-(2-hydroxy-2-(3-methoxy-4-hydroxyphenyl)-ethyl)-D(or L)-N-acetylglucosamine
2-deoxy-2-(2-(4-hydroxyphenyl)ethyl)-D(or L)-N-acetylglucosamine
2-deoxy-2-(2-methylamino-2-(4-hydroxyphenyl)ethyl)-D(or L)-N-acetylglucosamine
2-deoxy-2-(2-amino-2-(4-hydroxyphenyl)ethyl)-D(or L)-N-acetylglucosamine
2-deoxy-2-(2-hydroxy-2-(4-hydroxyphenyl)ethyl)-D(or L)-N-acetylgalactosamine
2-deoxy-2-(2-hydroxy-2-(4-methoxyphenyl)ethyl)-D(or L)-N-acetylgalactosamine
2-deoxy-2-(2-oxo-2-(4-hydroxyphenyl)ethyl)-D(or L)-N-acetylgalactosamine
2-deoxy-2-(2-oxo-2-(4-methoxyphenyl)ethyl)-D(or L)-N-acetylgalactosamine
2-deoxy-2-(2-hydroxy-2-(3-methoxy-4-hydroxyphenyl)-ethyl)-D(or L)-N-acetylgalactosamine
2-deoxy-2-(2-(4-hydroxyphenyl)ethyl)-D(or L)-N-acetylgalactosamine
2-deoxy-2-(2-methylamino-2-(4-hydroxyphenyl)ethyl)-D(or L)-N-acetylgalactosamine
2-deoxy-2-(2-amino-2-(4-hydroxyphenyl)ethyl)-D(or L)-N-acetylgalactosamine.

11. Composition according to any one of the preceding claims, **characterized in that** the compound of formula (I) is chosen from:
2-deoxy-2-(2-oxo-2-(4-hydroxyphenyl)ethyl)-D(or L)-glucose
2-deoxy-2-(2-hydroxy-2-(4-hydroxyphenyl)ethyl)-D(or L)-glucose
2-deoxy-2-(2-oxo-2-(4-methoxyphenyl)ethyl)-D(or L) - glucose
2-deoxy-2-(2-hydroxy-2-(4-methoxyphenyl)ethyl)-D(or L)-glucose.

12. Composition according to any one of the preceding claims, **characterized in that** the compound of formula (I) is present in an amount ranging from 0.001% to 10% by weight and preferably ranging from 0.01% to 5% by weight relative to the total weight of the composition.

13. Composition according to any one of the preceding claims, **characterized in that** it is intended for depigmenting and/or bleaching the skin.

14. Non therapeutical cosmetic process for depigmenting and/or bleaching human skin, body hairs or head hair, comprising the application to the skin, body hairs or head hair of a composition according to any one of the preceding claims.

15. Non therapeutical cosmetic use of a compound of formula (I) as defined according to any one of Claims 1 to 11, as an agent for bleaching and/or depigmenting the skin.

16. Use of a compound of formula (I) as defined according to any one of Claims 1 to 11, for the manufacture of a dermatological composition for depigmenting and/or bleaching the skin.

17. C-Glycoside compounds of formula (I'): 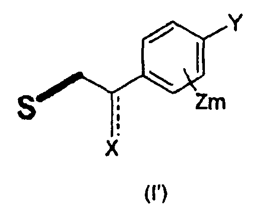 in which:
- S represents a monosaccharide or a polysaccharide containing up to 20 sugar units, in pyranose and/or furanose form and of L and/or D series, the said monosaccharide or polysaccharide containing at least one hydroxyl function that is necessarily free and/or optionally one or more optionally protected amine functions;
- the bond S-CH₂ being a C-glycoside bond;
- X represents a hydrogen atom or a group chosen from -OR₁ and -NR₁R₂ or an =O group;
- Y represents a group chosen from -OR₃, -NR₁R₂ and -OSi (R₄)₃;
- Z represents a fluorine atom or a group chosen from a linear C₁-C₆ alkyl group, a cyclic or branched C₃-C₆ alkyl, group, -OR₁, -NR₁R₂,- -COR₁, -CO₂R₁, -CONR₁R₂ and -CR₃;
- m is an integer ranging from 0 to 4;
with
- R₁ and R₂ denoting, independently of each other, a hydrogen atom or a linear C₁-C₆ alkyl group, a cyclic or branched C₃-C₆ alkyl group, aralkyl or phenyl;
- R₃ denoting a hydrogen atom or a linear C₁-C₆ alkyl group, a cyclic or branched C₃-C₆ alkyl group, aralkyl, or phenyl, or a group -SO₃H, -PO₃H₂ or -COR₁;
- R₄ denoting a linear C₁-C₆ alkyl group, a cyclic or branched C₃-C₆ alkyl group or phenyl;
with the proviso that when m = 0 and Y denotes an -OCH₃ group, S does not denote a D- (or L)-glucose, L-fucose, D- (or L)-xylose, or D- (or L)-maltose residue,
and also the cosmetically acceptable salts thereof, solvates thereof such as hydrates.

18. C-Glycoside compounds according to the receding claim, **characterized in that** S represents a monosaccharide or disaccharide, the hydroxyl function in position of which is free.

19. C-Glycoside compounds according to Claim 17 or 18, **characterized in that** S represents a monosaccharide chosen from D-glucose, D-galactose, D-mannose, D-fucose, L-fucose, D-xylose, D-lyxose, D-glucuronic acid, D-galacturonic acid, D-iduronic acid, N-acetyl-D-glucosamine and N-acetyl-D-galactosamine.

20. C-Glycoside compounds according to any one of Claims 17 to 19, **characterized in that** S represents D-glucose.

21. C-Glycoside compounds according to Claim 17 or 18, **characterized in that** S represents a polysaccharide containing up to 6 sugar units.

22. C-Glycoside compounds according to the preceding claim, **characterized in that** S represents a polysaccharide chosen from D-maltose, D-lactose, D-cellobiose, D-maltotriose, a disaccharide combining a uronic acid chosen from D-iduronic acid, D-glucuronic acid and D-galacturonic acid with a hexosamine chosen from D-galactosamine, D-glucosamine, N-acetyl-D-galactosamine, N-acetyl-D-glucosamine, an oligosaccharide containing at least one xylose.

23. C-Glycoside compounds according to Claim 21 or 22, **characterized in that** S represents a polysaccharide chosen from D-maltose and D-lactose.

24. C-Glycoside compounds according to any one of Claims 17 to 23, **characterized in that**:
- X denotes a group chosen from =O, -OH, -NH₂ and -NHCH₃;
- Z denotes a group chosen from -NH₂, -CH₃, -OH and -OCH₃;
- Y denotes a group -OR₃ as defined in Claim 17;
- m denotes an integer equal to 0, 1 or 2.

25. C-Glycoside compounds according to any one of Claims 17 to 24, **characterized in that**:
- S denotes a saccharide residue chosen from glucose, mannose, xylose, fucose, galactose, maltose, lactose, rhamnose, lyxose, arabinose, N-acetylglucosamine and N-acetylgalactosamine;
- X denotes a group chosen from =O, -OH, -NH₂ and -NHCH₃;
- Y denotes a group chosen from -OPO₃H₂, -OSO₃H, -OAc, -OH and -OCH₃;
- m = O.

26. C-Glycoside compounds according to any one of Claims 17 to 25, **characterized in that** the compound of formula (I') is chosen from:
2-deoxy-2-(2-hydroxy-2-(4-hydroxyphenyl)ethyl)-D(or L)-glucose
2-deoxy-2-(2-hydroxy-2-(4-hydroxyphenyl)ethyl)-D(or L)-galactose
2-deoxy-2-(2-hydroxy-2-(4-hydroxyphenyl)ethyl)-D(or L)-maltose
2-deoxy-2-(2-hydroxy-2-(4-hydroxyphenyl)ethyl)-D(or L)-mannose
2-deoxy-2-(2-hydroxy-2-(4-hydroxyphenyl)ethyl)-D(or L)-xylose
2-deoxy-2-(2-hydroxy-2-(4-hydroxyphenyl)ethyl)-D(or L)-fucose ,
2-deoxy-2-(2-hydroxy-2-(4-hydroxyphenyljethyl)-D(or L)-lactose
2-deoxy-2-(2-hydroxy-2-(4-hydroxyphenyl)ethyl)-D(or L)-rhamnose
2-deoxy-2-(2-hydroxy-2-(4-methoxyphenyl)ethyl)-D(or L)-glucose
2-deoxy-2-(2-hydroxy-2-(4-methoxyphenyl)ethyl)-D(or L)-galactose
2-deoxy-2-(2-hydroxy-2-(4-methoxyphenyl)ethyl)-D(or L)-maltose
2-deoxy-2-(2-hydroxy-2-(4-methoxyphenyl)ethyl)-D(or L)-mannose
2-deoxy-2-(2-hydroxy-2-(4-methoxyphenyl)ethyl)-D(or L)-xylose
2-deoxy-2-(2-hydroxy-2-(4-methoxyphenyl)ethyl)-D(or L)-fucose
2-deoxy-2-(2-hydroxy-2-(4-methoxyphenyl)ethyl)-D(or L)-lactose
2-deoxy-2-(2-hydroxy-2-(4-methoxyphenyl)ethyl)-D(or L)-rhamnose
2-deoxy-2-(2-oxo-2-(4-hydroxyphenyl)ethyl)-D(or L)-glucose
2-deoxy-2-(2-oxo-2-(4-hydroxyphenyl)ethyl)-D(or L)-galactose
2-deoxy-2-(2-oxo-2-(4-hydroxyphenyl)ethyl)-D(or L)-maltose
2-deoxy-2-(2-oxo-2-(4-hydroxyphenyl)ethyl)-D(or L)-mannose
2-deoxy-2-(2-oxo-2-(4-hydroxyphenyl)ethyl)-D(or L)-xylose
2-deoxy-2-(2-oxo-2-(4-hydroxyphenyl)ethyl)-D(or L)-fucose
2-deoxy-2-(2-oxo-2-(4-hydroxyphenyl)ethyl)-D(or L)-lactose
2-deoxy-2-(2-oxo-2-(4-hydroxyphenyl)ethyl)-D(or L)-rhamnose
2-deoxy-2-(2-oxo-2-(4-methoxyphenyl)ethyl)-D(or L)-glucose
2-deoxy-2-(2-oxo-2-(4-methoxyphenyl)ethyl)-D(or L)-galactose
2-deoxy-2-(2-oxo-2-(4-methoxyphenyl)ethyl)-D(or L)-maltose
2-deoxy-2-(2-oxo-2-(4-methoxyphenyl)ethyl)-D(or L)-mannose
2-deoxy-2-(2-oxo-2-(4-methoxyphenyl)ethyl)-D(or L)-xylose
2-deoxy-2-(2-oxo-2-(4-methoxyphenyl)ethyl)-D(or L)-fucose
2-deoxy-2-(2-oxo-2-(4-methoxyphenyl)ethyl)-D(or L)-lactose
2-deoxy-2-(2-oxo-2-(4-methoxyphenyl)ethyl)-D(or L)-rhamnose
2-deoxy-2-(2-hydroxy-2-(3-methoxy-4-hydroxyphenyl)-ethyl)-D(or L)-glucose
2-deoxy-2-(2-hydroxy-2-(3-methoxy-4-hydroxyphenyl)-ethyl)-D(or L)-galactose
2-deoxy-2-(2-hydroxy-2-(3-methoxy-4-hydroxyphenyl)-ethyl)-D(or L)-maltose
2-deoxy-2-(2-hydroxy-2-(3-methoxy-4-hydroxyphenyl)-ethyl)-D(or L)-mannose
2-deoxy-2-(2-hydroxy-2-(3-methoxy-4-hydroxyphenyl)-ethyl)-D(or L)-xylose
2-deoxy-2-(2-hydroxy-2-(3-methoxy-4-hydroxyphenyl)-ethyl)-D(or L)-fucose
2-deoxy-2-(2-hydroxy-2-(3-methoxy-4-hydroxyphenyl)-ethyl)-D(or L)-lactose
2-deoxy-2-(2-hydroxy-2-(3-methoxy-4-hydroxyphenyl)-ethyl)-D(or L)-rhamnose
2-deoxy-2-(2-(4-hydroxyphenyl)ethyl)-D(or L)-glucose
2-deoxy-2-(2-(4-hydroxyphenyl)ethyl)-D(or L)-galactose
2-deoxy-2-(2-(4-hydroxyphenyl)ethyl)-D(or L)-maltose
2-deoxy-2-(2-(4-hydroxyphenyl)ethyl)-D(or L)-mannose
2-deoxy-2-(2-(4-hydroxyphenyl)ethyl)-D(or L)-xylose
2-deoxy-2-(2-(4-hydroxyphenyl)ethyl)-D(or L)-fucose
2-deoxy-2-(2-(4-hydroxyphenyl)ethyl)-D(or L)-lactose
2-deoxy-2-(2-(4-hydroxyphenyl)ethyl)-D(or L)-rhamnose
2-deoxy-2-(2-methylamino-2-(4-hydroxyphenyl)ethyl)-D(or L)-glucose
2-deoxy-2-(2-methylamino-2-(4-hydroxyphenyl)ethyl)-D(or L)-galactose
2-deoxy-2-(2-methylamino-2-(4-hydroxyphenyl)ethyl)-D(or L)-maltose
2-deoxy-2-(2-methylamino-2-(4-hydroxyphenyl)ethyl)-D(or L)-mannose
2-deoxy-2-(2-methylamino-2-(4-hydroxyphenyl)ethyl)-D(or L)-xylose
2-deoxy-2-(2-methylamino-2-(4-hydroxyphenyl)ethyl)-D(or L)-fucose
2-deoxy-2-(2-methylamino-2-(4-hydroxyphenyl)ethyl)-D(or L)-lactose
2-deoxy-2-(2-methylamino-2-(4-hydroxyphenyl)ethyl)-D(or L)-rhamnose
2-deoxy-2-(2-amino-2-(4-hydroxyphenyl)ethyl)-D(or L)-glucose
2-deoxy-2-(2-amino-2-(4-hydroxyphenyl)ethyl)-D(or L)-galactose
2-deoxy-2-(2-amino-2-(4-hydroxyphenyl)ethyl)-D(or L)-maltose
2-deoxy-2-(2-amino-2-(4-hydroxyphenyl)ethyl)-D(or L)-mannose
2-deoxy-2-(2-amino-2-(4-hydroxyphenyl)ethyl)-D(or L)-xylose
2-deoxy-2-(2-amino-2-(4-hydroxyphenyl)ethyl)-D(or L)-fucose
2-deoxy-2-(2-amino-2-(4-hydroxyphenyl)ethyl)-D(or L)-lactose
2-deoxy-2-(2-amino-2-(4-hydroxyphenyl)ethyl)-D(or L)-rhamnose
2-deoxy-2-(2-hydroxy-2-(4-hydroxyphenyl)ethyl)-D(or L)-lyxose
2-deoxy-2-(2-hydroxy-2-(4-methoxyphenyl)ethyl)-D(or L)-lyxose
2-deoxy-2-(2-oxo-2-(9-hydroxyphenyl)ethyl)-D(or L)-lyxose
2-deoxy-2-(2-oxo-2-(4-methoxyphenyl)ethyl)-D(or L)-lyxose
2-deoxy-2-(2-hydroxy-2-(3-methoxy-4-hydroxyphenyl)-ethyl)-D(or L)-lyxose
2-deoxy-2-(2-(4-hydroxyphenyl)ethyl)-D(or L)-lyxose
2-deoxy-2-(2-methylamino-2-(4-hydroxyphenyl)ethyl)-D(or L)-lyxose
2-deoxy-2-(2-amino-2-(4-hydroxyphenyl)ethyl)-D(or L)-lyxose
2-deoxy-2-(2-hydroxy-2-(9-hydroxyphenyl)ethyl)-D(or L)-arabinose
2-deoxy-2-(2-hydroxy-2-(4-methoxyphenyl)ethyl)-D(or L)-arabinose
2-deoxy-2-(2-oxo-2-(4-hydroxyphenyl)ethyl)-D(or L)-arabinose
2-deoxy-2-(2-oxo-2-(4-methoxyphenyl)ethyl)-D(or L)-arabinose
2-deoxy-2-(2-hydroxy-2-(3-methoxy-4-hydroxyphenyl)-ethyl)-D(or L)-arabinose
2-deoxy-2-(2-(4-hydroxyphenyl)ethyl)-D(or L)-arabinose
2-deoxy-2-(2-methylamino-2-(4-hydroxyphenyl)ethyl)-D(or L)-arabinose
2-deoxy-2-(2-amino-2-(4-hydroxyphenyl)ethyl)-D(or L)-arabinose
2-deoxy-2-(2-hydroxy-2-(4-hydroxyphenyl)ethyl)-D(or L)-N-acetylglucosamine
2-deoxy-2-(2-hydroxy-2-(4-methoxyphenyl)ethyl)-D(or L)-N-acetylglucosamine
2-deoxy-2-(2-oxo-2-(4-hydroxyphenyl)ethyl)-D(or L)-N-acetylglucosamine
2-deoxy-2-(2-oxo-2-(4-methoxyphenyl) ethyl)-D(or L)-N-acetylglucosamine
2-deoxy-2-(2-hydroxy-2-(3-methoxy-4-hydroxyphenyl)-ethyl)-D(or L)-N-acetylglucosamine
2-deoxy-2-(2-(4-hydroxyphenyl) ethyl)-D(or L)-N-acetylglucosamine.
2-deoxy-2-(2-methylamino-2-(4-hydroxyphenyl)ethyl)-D(or L)-N-acetylglucosamine
2-deoxy-2-(2-amino-2-(4-hydroxyphenyl)ethyl)-D(or L)-N-acetylglucosamine
2-deoxy-2-(2-hydroxy-2-(4-hydroxyphenyl)ethyl)-D(or L)-N-acetylgalactosamine
2-deoxy-2-(2-hydroxy-2-(4-methoxyphenyl)ethyl)-D(or L)-N-acetylgalactosamine
2-deoxy-2-(2-oxo-2-(4-hydroxyphenyl)ethyl)-D(or L)-N-acetylgalactosamine
2-deoxy-2-(2-oxo-2-(4-methoxyphenyl)ethyl)-D(or L)-N-acetylgalactosamine
2-deoxy-2-(2-hydroxy-2-(3-methoxy-4-hydroxyphenyl)-ethyl)-D(or L)-N-acetylgalactosamine
2-deoxy-2-(2-(4-hydroxyphenyl)ethyl)-D(or L)-N-acetylgalactosamine
2-deoxy-2-(2-methylamino-2-(4-hydroxyphenyl)ethyl)-D(or L)-N-acetylgalactosamine
2-deoxy-2-(2-amino-2-(4-hydrox.yphenyl)ethyl)-D(or L)-N-acetylgalactosamine.

27. C-Glycoside compounds according to any one of Claims 17 to 26, **characterized in that** the compound of formula (I') is chosen from:
2-deoxy-2-(2-oxo-2-(4-hydroxyphenyl)ethyl)-D(or L)-glucose
2-deoxy-2-(2-hydroxy-2-(4-hydroxyphenyl)ethyl)-D(or L)-glucose
2-deoxy-2-(2-oxo-2-(4-methoxyphenyl)ethyl)-D(or L)-glucose
2-deoxy-2-(2-hydroxy-2-(4-methoxyphenyl)ethyl)-D(or L)-glucose.

## Patentansprüche

1. Zusammensetzung, die in einem physiologisch akzeptablen Medium mindestens eine Verbindung der Formel (I): 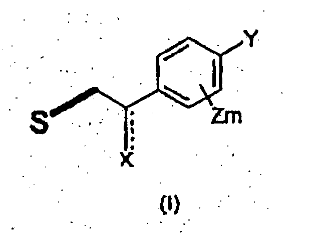 in der Formel:
- S bedeutet ein Monosaccharid oder ein Polysaccharid mit bis zu 20 Zuckereinheiten in Pyranose- und/oder Furanoseform und der L- und/oder D-Reihe, wobei das Monosaccharid oder Polysaccharid mindestens eine Hydroxyfunktion, die zwingend frei vorliegt, und/ oder gegebenenfalls eine oder mehrere geschützte Aminfunktionen enthält,
- wobei die Bindung S-CH₂ eine C-Glycosid-Bindung ist,
- X bedeutet ein Wasserstoffatom oder eine Gruppe, die unter -OR₁ und -NR₁R₂ oder einer Gruppe =O ausgewählt ist,
- Y bedeutet eine Gruppe, die unter -OR₃, -NR₁R₂ und -OSi(R₄)₃ ausgewählt ist,
- Z bedeutet ein Fluoratom oder eine Gruppe, die unter einer geradkettigen C₁₋₆-Alkylgruppe, einer cyclischen oder einer verzweigten C₃-₆-Alkylgruppe, -OR₁, -NR₁R₂, -COR₁, -CO₂R₁, -CONR₁R₂ und -CR₃ ausgewählt ist,
- m ist Null oder eine ganze Zahl von 1 bis 4, wobei
- R₁ und R₂ unabhängig voneinander ein Wasserstoffatom oder eine geradkettige C₁₋₆-Alkylgruppe, eine cyclische oder verzweigte C₃₋₆-Alkylgruppe, Aralkyl oder Phenyl bedeuten,
- R₃ ein Wasserstoffatom oder eine geradkettige C₁₋₆-Alkylgruppe, eine cyclische oder verzweigte C₃₋₆-Alkylgruppe, Aralkyl oder Phenyl oder eine Gruppe -SO₃H, -PO₃H₂ oder -COR₁ bedeutet,
- R₄ eine geradkettige C₁₋₆-Alkylgruppe, eine cyclische oder verzweigte C₃₋₆-Alkylgruppe oder Phenyl bedeutet,
und auch deren kosmetisch akzeptablen Salze und deren Solvate wie Hydrate;
und einen Bestandteil enthält, der unter Wasser, Ölen mineralischer, tierischer und/oder pflanzlicher Herkunft, Wachsen, Pigmenten, Füllstoffen, grenzflächenaktiven Stoffen, UV-Filtern, Polymeren und Quellmitteln ausgewählt ist.

2. Zusammensetzung nach dem vorherigen Anspruch, **dadurch gekennzeichnet, dass** S ein Monosaccharid oder Disaccharid bedeutet, wobei die Hydroxyfunktion in 3-Stellung frei vorliegt.

3. Zusammensetzung nach einem der vorhergehenden Ansprüche, **dadurch gekennzeichnet, dass** S ein Monosaccharid bedeutet, das unter D-Glucose, D-Galactose, D-Mannose, D-Fucose, L-Fucose, D-Xylose, D-Lyxose, D-Glucuronsäure, D-Galacturonsäure, D-Iduronsäure, N-Acetyl-D-glucosamin und N-Acetyl-D-galactosamin ausgewählt ist.

4. Zusammensetzung nach einem der vorhergehenden Ansprüche, **dadurch gekennzeichnet, dass** S die D-Glucose bedeutet.

5. Zusammensetzung nach Anspruch 1 oder 2, **dadurch gekennzeichnet, dass** S ein Polysaccharid bedeutet, dass bis zu 6 Zuckereinheiten aufweist.

6. Zusammensetzung nach einem der Ansprüche 1, 2 und 5, **dadurch gekennzeichnet, dass** S ein Polysaccharid bedeutet, das unter D-Maltose, D-Lactose, D-Cellobiose, D-Maltotriose, einem Disaccharid, bei dem eine Uronsäure, die unter D-Iduronsäure, D-Glucuronsäure und D-Galacturonsäure ausgewählt ist, mit einem Hexosamin kombiniert ist, das unter D-Galactosamin, D-Glucosamin, N-Acetyl-D-galactosamin, N-Acetyl-D-glucosamin, einem Oligosaccharid, das mindestens eine Xylose enthält, ausgewählt ist.

7. Zusammensetzung nach einem der Ansprüche 1, 2, 5 und 6, **dadurch gekennzeichnet, dass** S ein Polysaccharid bedeutet, das unter D-Maltose und D-Lactose ausgewählt ist.

8. Zusammensetzung nach einem der vorhergehenden Ansprüche,
**dadurch gekennzeichnet, dass**:
- X eine Gruppe bedeutet, die unter =O, -OH, -NH₂ und
-NHCH₃ ausgewählt ist;
- Z eine Gruppe bedeutet, die unter -NH₂, -CH₃, -OH und
-OCH₃ ausgewählt ist;
- Y eine Gruppe -OR₃ bedeutet, wie sie in Anspruch 1 definiert ist;
- m eine ganze Zahl gleich 0, 1 oder 2 ist.

9. Zusammensetzung nach einem der vorhergehenden Ansprüche,
**dadurch gekennzeichnet, dass**:
- S einen Saccharidrest bedeutet, der unter Glucose, Mannose, Xylose, Fucose, Galactose, Maltose, Lactose, Rhamnose, Lyxose, Arabinose, N-Acetylglucosamin und N-Acetylgalactosamin ausgewählt ist;
- X eine Gruppe bedeutet, die unter =O, -OH, -NH₂ und
-NHCH₃ ausgewählt ist;
- Y eine Gruppe bedeutet, die unter -PO₃H₂, -OSO₃H, -OAc,
-OH und -OCH₃ ausgewählt ist;
- m=0.

10. Zusammensetzung nach einem der vorhergehenden Ansprüche, **dadurch gekennzeichnet, dass** die Verbindung der Formel (I) ausgewählt ist unter:
2-Deoxy-2-(2-hydroxy-2-(4-hydroxyphenyl)ethyl)-D(oder L)-glucose
2-Deoxy-2-(2-hydroxy-2-(4-hydroxyphenyl)ethyl)-D(oder L)-galactose
2-Deoxy-2-(2-hydroxy-2-(4-hydroxyphenyl)ethyl)-D(oder L)-maltose
2-Deoxy-2-(2-hydroxy-2-(4-hydroxyphenyl)ethyl)-D(oder L)-mannose
2-Deoxy-2-(2-hydroxy-2-(4-hydroxyphenyl)ethyl)-D(oder L)-xylose
2-Deoxy-2-(2-hydroxy-2-(4-hydroxyphenyl)ethyl)-D(oder L)-fucose
2-Deoxy-2-(2-hydroxy-2-(4-hydroxyphenyl)ethyl)-D(oder L)-lactose
2-Deoxy-2-(2-hydroxy-2-(4-hydroxyphenyl)ethyl)-D(oder L)-rhamnose
2-Deoxy-2-(2-hydroxy-2-(4-methoxyphenyl)ethyl)-D(oder L)-glucose
2-Deoxy-2-(2-hydroxy-2-(4-methoxyphenyl)ethyl)-D(oder L)-galactose
2-Deoxy-2-(2-hydroxy-2-(4-methoxyphenyl)ethyl)-D(oder L)-maltose
2-Deoxy-2-(2-hydroxy-2-(4-methoxyphenyl)ethyl)-D(oder L)-mannose
2-Deoxy-2-(2-hydroxy-2-(4-methoxyphenyl)ethyl)-D(oder L)-xylose
2-Deoxy-2-(2-hydroxy-2-(4-methoxyphenyl)ethyl)-D(oder L)-fucose
2-Deoxy-2-(2-hydroxy-2-(4-methoxyphenyl)ethyl)-D(oder L)-lactose
2-Deoxy-2-(2-hydroxy-2-(4-methoxyphenyl)ethyl)-D(oder L)-rhamnose
2-Deoxy-2-(2-oxo-2-(4-hydroxyphenyl)ethyl)-D(oder L)-glucose
2-Deoxy-2-(2-oxo-2-(4-hydroxyphenyl)ethyl)-D(oder L)-galactose
2-Deoxy-2-(2-oxo-2-(4-hydroxyphenyl)ethyl)-D(oder L)-maltose
2-Deoxy-2-(2-oxo-2-(4-hydroxyphenyl)ethyl)-D(oder L)-mannose
2-Deoxy-2-(2-oxo-2-(4-hydroxyphenyl)ethyl)-D(oder L)-xylose
2-Deoxy-2-(2-oxo-2-(4-hydroxyphenyl)ethyl)-D(oder L)-fucose
2-Deoxy-2-(2-oxo-2-(4-hydroxyphenyl)ethyl)-D(oder L)-lactose
2-Deoxy-2-(2-oxo-2-(4-hydroxyphenyl)ethyl)-D(oder L)-rhamnose
2-Deoxy-2-(2-oxo-2-(4-methoxyphenyl)ethyl)-D(oder L)-glucose
2-Deoxy-2-(2-oxo-2-(4-methoxyphenyl)ethyl)-D(oder L)-galactose
2-Deoxy-2-(2-oxo-2-(4-methoxyphenyl)ethyl)-D(oder L)-maltose
2-Deoxy-2-(2-oxo-2-(4-methoxyphenyl)ethyl)-D(oder L)-mannose
2-Deoxy-2-(2-oxo-2-(4-methoxyphenyl)ethyl)-D(oder L)-xylose
2-Deoxy-2-(2-oxo-2-(4-methoxyphenyl)ethyl)-D(oder L)-fucose
2-Deoxy-2-(2-oxo-2-(4-methoxyphenyl)ethyl)-D(oder L)-lactose
2-Deoxy-2-(2-oxo-2-(4-methoxyphenyl)ethyl)-D(oder L)-rhamnose
2-Deoxy-2-(2-hydroxy-2-(3-methoxy-4-hydroxyphenyl)ethyl)-D(oder L)-glucose
2-Deoxy-2-(2-hydroxy-2-(3-methoxy-4-hydroxyphenyl)ethyl)-D(oder L)-galactose
2-Deoxy-2-(2-hydroxy-2-(3-methoxy-4-hydroxyphenyl)ethyl)-D(oder L)-maltose
2-Deoxy-2-(2-hydroxy-2-(3-methoxy-4-hydroxyphenyl)ethyl)-D(oder L)-mannose
2-Deoxy-2-(2-hydroxy-2-(3-methoxy-4-hydroxyphenyl)ethyl)-D(oder L)-xylose
2-Deoxy-2-(2-hydroxy-2-(3-methoxy-4-hydroxyphenyl)ethyl)-D(oder L)-fucose
2-Deoxy-2-(2-hydroxy-2-(3-methoxy-4-hydroxyphenyl)ethyl)-D(oder L)-lactose
2-Deoxy-2-(2-hydroxy-2-(3-methoxy-4-hydroxyphenyl)ethyl)-D(oder L)-rhamnose
2-Deoxy-2-(2-(4-hydroxyphenyl)ethyl)-D(oder L)-glucose
2-Deoxy-2-(2-(4-hydroxyphenyl)ethyl)-D(oder L)-galactose
2-Deoxy-2-(2-(4-hydroxyphenyl)ethyl)-D(oder L)-maltose
2-Deoxy-2-(2-(4-hydroxyphenyl)ethyl)-D(oder L)-mannose
2-Deoxy-2-(2-(4-hydroxyphenyl)ethyl)-D(oder L)-xylose
2-Deoxy-2-(2-(4-hydroxyphenyl)ethyl)-D(oder L)-fucose
2-Deoxy-2-(2-(4-hydroxyphenyl)ethyl)-D(oder L)-lactose
2-Deoxy-2-(2-(4-hydroxyphenyl)ethyl)-D(oder L)-rhamnose
2-Deoxy-2-(2-methylamino-2-(4-hydroxyphenyl)ethyl)-D(oder L)-glucose
2-Deoxy-2-(2-methylamino-2-(4-hydroxyphenyl)ethyl)-D(oder L)-galactose
2-Deoxy-2-(2-methylamino-2-(4-hydroxyphenyl)ethyl)-D(oder L)-maltose
2-Deoxy-2-(2-methylamino-2-(4-hydroxyphenyl)ethyl)-D(oder L)-mannose
2-Deoxy-2-(2-methylamino-2-(4-hydroxyphenyl)ethyl)-D(oder L)-xylose
2-Deoxy-2-(2-methylamino-2-(4-hydroxyphenyl)ethyl)-D(oder L)-fucose
2-Deoxy-2-(2-methylamino-2-(4-hydroxyphenyl)ethyl)-D(oder L)-lactose
2-Deoxy-2₋(2-methylamino-2-(4-hydroxyphenyl)ethyl)-D(oder L)-rhamnose
2-Deoxy-2-(2-amino-2-(4-hydroxyphenyl)ethyl)-D(oder L)-glucose
2-Deoxy-2-(2-amino-2-(4-hy.droxyphenyl)ethyl)-D(oder L)-galactose
2-Deoxy-2-(2-amino-2-(4-hydroxyphenyl)ethyl)-D(oder L)-maltose
2-Deoxy-2-(2-amino-2-(4-hydroxyphenyl)ethyl)-D(oder L)-mannose
2-Deoxy-2-(2-amino-2-(4-hydroxyphenyl)ethyl)-D(oder L)-xylose
2-Deoxy-2-(2-amino-2-(4-hydroxyphenyl)ethyl)-D(oder L)-fucose
2-Deoxy-2-(2-amino-2-(4-hydroxyphenyl)ethyl)-D(oder L)-lactose
2-Deoxy-2-(2-amino-2-(4-hydroxyphenyl)ethyl)-D(oder L)-rhamnose
2-Deoxy-2-(2-hydroxy-2-(4-hydroxyphenyl)ethyl)-D(oder L)-lyxose
2-Deoxy-2-(2-hydroxy-2-(4-methoxyphenyl)ethyl)-D(oder L)-lyxose
2-Deoxy-2-(2-oxo-2-(4-hydroxyphenyl)ethyl)-D(oder L)-lyxose
2-Deoxy-2-(2-oxo-2-(4-methoxyphenyl)ethyl)-D(oder L)-lyxose
2-Deoxy-2-(2-hydroxy-2-(3-methoxy-4-hydroxyphenyl)ethyl)-D(oder L)-lyxose
2-Deoxy-2-(2-(4-hydroxyphenyl)ethyl)-D(oder L)-lyxose
2-Deoxy-2-(2-methylamino-2-(4-hydroxyphenyl)ethyl)-D(oder L)-lyxose
2-Deoxy-2-(2-amino-2-(4-hydroxyphenyl)ethyl)-D(oder L)-lyxose
2-Deoxy-2-(2-hydroxy-2-(4-hydroxyphenyl)ethyl)-D(oder L)-arabinose
2-Deoxy-2-(2-hydroxy-2-(4-methoxyphenyl)ethyl)-D(oder L)-arabinose
2-Deoxy-2-(2-oxo-2-(4-hydroxyphenyl)ethyl)-D(oder L)-arabinose
2-Deoxy-2-(2-oxo-2-(4-methoxyphenyl)ethyl)-D(oder L)-arabinose
2-Deoxy-2-(2-hydroxy-2-(3-methoxy-4-hydroxyphenyl)ethyl)-D(oder L)-arabinose
2-Deoxy-2-(2-(4-hydroxyphenyl)ethyl)-D(oder L)-arabinose
2-Deoxy-2-(2-methylamino-2-(4-hydroxyphenyl)ethyl)-D(oder L)-arabinose
2-Deoxy-2-(2-amino-2-(4-hydroxyphenyl)ethyl)-D(oder L)-arabinose
2-Deoxy-2-(2-hydroxy-2-(4-hydroxyphenyl)ethyl)-D(oder L)-N-acetylglucosamin
2-Deoxy-2-(2-hydroxy-2-(4-methoxyphenyl)ethyl)-D(oder L)-N-acetylglucosamin
2-Deoxy-2-(2-oxo-2-(4-hydroxyphenyl)ethyl)-D(oder L)-N-acetylglucosamin
2-Deoxy-2-(2-oxo-2-(4-methoxyphenyl)ethyl)-D(oder L)-N-acetylglucosamin
2-Deoxy-2-(2-hydroxy-2-(3-methoxy-4-hydroxyphenyl)ethyl)-D(oder L)-N-acetylglucosamin
2-Deoxy-2-(2-(4-hydroxyphenyl)ethyl)-D(oder L)-N-acetylglucosamin
2-Deoxy-2-(2-methylamino-2-(4-hydroxyphenyl)ethyl)-D(oder L)-N-acetylglucosamin
2-Deoxy-2-(2-amino-2-(4-hydroxyphenyl)ethyl)-D(oder L)-N-acetylglucosamin
2-Deoxy-2-(2-hydroxy-2-(4-hydroxyphenyl)ethyl)-D(oder L)-N-acetylgalactosamin
2-Deoxy-2-(2-hydroxy-2-(4-methoxyphenyl)ethyl)-D(oder L)-N-acetylgalactosamin
2-Deoxy-2-(2-oxo-2-(4-hydroxyphenyl)ethyl)-D(oder L)-N-acetylgalactosamin
2-Deoxy-2-(2-oxo-2-(4-methoxyphenyl)ethyl)-D(oder L)-N-acetylgalactosamin
2-Deoxy-2-(2-hydroxy-2-(3-methoxy-4-hydroxyphenyl)ethyl)-D(oder L)-N-acetylgalactosamin
2-Deoxy-2-(2-(4-hydroxyphenyl)ethyl)-D(oder L)-N-acetylgalactosamin
2-Deoxy-2-(2-methylamino-2-(4-hydroxyphenyl)ethyl)-D(oder L)-N-acetylgalactosamin
2-Deoxy-2-(2-amino-2-(4-hydroxyphenyl)ethyl)-D(oder L)-N-acetylgalactosamin.

11. Zusammensetzung nach einem der vorhergehenden Ansprüche, **dadurch gekennzeichnet, dass** die Verbindung der Formel (I) ausgewählt ist unter:
2-Deoxy-2-(2-oxo-2-(4-hydroxyphenyl)ethyl)-D(oder L)-glucose
2-Deoxy-2-(2-hydroxy-2-(4-hydroxyphenyl)ethyl)-D(oder L)-glucose
2-Deoxy-2-(2-oxo₋2-(4-methoxyphenyl)ethyl)-D(oder L)-glucose
2-Deoxy-2-(2-hydroxy-2-(4-methoxyphenyl)ethyl)-D(oder L)-glucose.

12. Zusammensetzung nach einem der vorhergehenden Ansprüche, **dadurch gekennzeichnet, dass** die Verbindung der Formel (I) in einer Menge von 0,001 bis 10 Gew.-% und vorzugsweise 0,01 bis 5 Gew.-%, bezogen auf das Gesamtgewicht der Zusammensetzung, vorliegt.

13. Zusammensetzung nach einem der vorhergehenden Ansprüche, **dadurch gekennzeichnet, dass** sie zum Depigmentieren und/oder Bleichen der Haut vorgesehen ist.

14. Nicht therapeutisches kosmetisches Verfahren zum Depigmentieren und/oder Bleichen menschlicher Haut, von Körperhaar oder Kopfhaar, das das Auftragen einer Zusammensetzung nach einem der vorhergehenden Ansprüche auf die Haut, die Körperhaare oder die Kopfhaare umfasst.

15. Nicht therapeutische kosmetische Verwendung einer Verbindung der Formel (I), wie sie in einem der Ansprüche 1 bis 11 definiert ist, als Mittel zum Bleichen und/oder Depigmentieren der Haut.

16. Verwendung einer Verbindung der Formel (I), wie sie in einem der Ansprüche 1 bis 11 definiert ist, für die Herstellung einer dermatologischen Zusammensetzung zum Depigmentieren und/oder Bleichen der Haut.

17. C-Glycosidverbindungen der Formel (I'): 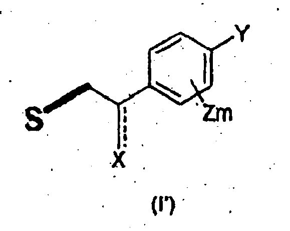 in der Formel:
- S bedeutet ein Monosaccharid oder ein Polysaccharid mit bis zu 20 Zuckereinheiten in Pyranose- und/oder Furanoseform und der L- und/oder D-Reihe, wobei das Monosaccharid oder Polysaccharid mindestens eine Hydroxyfunktion, die zwingend frei vorliegt, und/oder gegebenenfalls eine oder mehrere geschützte Aminfunktionen enthält,
- wobei die Bindung S-CH₂ eine C-Glycosid-Bindung ist,
- X bedeutet ein Wasserstoffatom oder eine Gruppe, die unter -OR₁ und -NR₁R₂ oder einer Gruppe =O ausgewählt ist,
- Y bedeutet eine Gruppe, die unter -OR₃, -NR₁R₂ und -OSi(R₄)₃ ausgewählt ist,
- Z bedeutet ein Fluoratom oder eine Gruppe, die unter einer geradkettigen C₁₋₆-Alkylgruppe, einer cyclischen oder einer verzweigten C₃₋₆-Alkylgruppe, -OR₁, -NR₁R₂, -COR₁, -CO₂R₁, -CONR₁R₂ und -CR₃ ausgewählt ist,
- m ist Null oder eine ganze Zahl von 1 bis 4,
wobei
- R₁ und R₂ unabhängig voneinander ein Wasserstoffatom oder eine geradkettige C₁₋₆-Alkylgruppe, eine cyclische oder verzweigte C₃₋₆-Alkylgruppe, Aralkyl oder Phenyl bedeuten,
- R₃ ein Wasserstoffatom oder eine geradkettige C₁₋₆-Alkylgruppe, eine cyclische oder verzweigte C₃₋₆-Alkylgruppe, Aralkyl oder Phenyl oder eine Gruppe -SO₃H, -PO₃H₂ oder -COR₁ bedeutet,
- R₄ eine geradkettige C₁₋₆-Alkylgruppe, eine cyclische oder verzweigte C₃₋₆-Alkylgruppe oder Phenyl bedeutet, mit der Maßgabe, dass S keinen D-(oder L)- Glucoserest, L-Fucoserest, D-(oder L)- Xyloserest oder D-(oder L)- Maltoserest bedeutet;
und auch deren kosmetisch akzeptablen Salze und deren Solvate wie Hydrate;

18. C-Glycosidverbindungen nach dem vorherigen Anspruch, **dadurch gekennzeichnet, dass** S ein Monosaccharid oder Disaccharid bedeutet, wobei die Hydroxyfunktion in 3-Stellung frei vorliegt.

19. C-Glycosidverbindungen nach Anspruch 17 oder 18, **dadurch gekennzeichnet, dass** S ein Monosaccharid bedeutet, das unter D-Glucose, D-Galactose, D-Mannose, D-Fucose, L-Fucose, D-Xylose, D-Lyxose, D-Glucuronsäure, D-Galacturonsäure, D-Iduronsäure, N-Acetyl-D-Glucosamin und N-Acetyl-D-galactosamin ausgewählt ist.

20. C-Glycosidverbindungen nach einem der Ansprüche 17 bis 19, **dadurch gekennzeichnet, dass** S D-Glucose bedeutet.

21. C-Glycosidverbindungen nach Anspruch 17 oder 18, **dadurch gekennzeichnet, dass** S ein Polysaccharid bedeutet, dass bis zu 6 Zuckereinheiten aufweist.

22. C-Glycosidverbindungen nach dem vorhergehenden Anspruch, **dadurch gekennzeichnet, dass** S ein Polysaccharid bedeutet, das unter D-Maltose, D-Lactose, D-Cellobiose, D-Maltotriose, einem Disaccharid, bei dem eine Uronsäure, die unter D-Iduronsäure, D-Glucuronsäure und D-Galacturonsäure ausgewählt ist, mit einem Hexosamin kombiniert ist, das unter D-Galactosamin, D-Glucosamin, N-Acetyl-D-galactosamin, N-Acetyl-D-glucosamin, einem Oligosaccharid, das mindestens eine Xylose enthält, ausgewählt ist.

23. C-Glycosidverbindungen nach Anspruch 21 oder 22, **dadurch gekennzeichnet, dass** S ein Polysaccharid bedeutet, das unter D-Maltose und D-Lactose ausgewählt ist.

24. C-Glycosidverbindungen nach einem der Ansprüche 17 bis 23,
**dadurch gekennzeichnet, dass**:
- X eine Gruppe bedeutet, die unter =O, -OH, -NH₂ und
-NHCH₃ ausgewählt ist;
- Z eine Gruppe bedeutet, die unter -NH₂, -CH₃, -OH und
-OCH₃ ausgewählt ist;
- Y eine Gruppe -OR₃ bedeutet, wie sie in Anspruch 17 definiert ist;
- m eine ganze Zahl gleich 0, 1 oder 2 ist.

25. C-Glycosidverbindungen nach einem der Ansprüche 17 bis 24,
**dadurch gekennzeichnet, dass**:
- S einen Saccharidrest bedeutet, der unter Glucose, Mannose, Xylose, Fucose, Galactose, Maltose, Lactose, Rhamnose, Lyxose, Arabinose, N-Acetylglucosamin und N-Acetylgalactosamin ausgewählt ist;
- X eine Gruppe bedeutet, die unter =O, -OH, -NH₂ und
-NHCH₃ ausgewählt ist;
- Y eine Gruppe bedeutet, die unter -PO₃H₂, -OSO₃H, -OAc, -
OH und -OCH₃ ausgewählt ist;
- m = 0.

26. C-Glycosidverbindungen nach einem der Ansprüche 17 bis 25, **dadurch gekennzeichnet, dass** die Verbindung der Formel (I') ausgewählt ist unter:
2-Deoxy-2-(2-hydroxy-2-(4-hydroxyphenyl)ethyl)-D(oder L)-glucose
2-Deoxy-2-(2-hydroxy-2-(4-hydroxyphenyl)ethyl)-D(oder L)-galactose
2-Deoxy-2-(2-hydroxy-2-(4-hydroxyphenyl)ethyl)-D(oder L)-maltose
2-Deoxy-2-(2-hydroxy-2-(4-hydroxyphenyl)ethyl)-D(oder L)-mannose
2-Deoxy-2-(2-hydroxy-2-(4-hydroxyphenyl)ethyl)-D(oder L)-xylose
2-Deoxy-2-(2-hydroxy-2-(4-hydroxyphenyl)ethyl)-D(oder L)-fucose
2-Deoxy-2-(2-hydroxy-2-(4-hydroxyphenyl)ethyl)-D(oder L)-lactose
2-Deoxy-2-(2-hydroxy-2-(4-hydroxyphenyl)ethyl)-D(oder L)-rhamnose
2-Deoxy-2-(2-hydroxy-2-(4-methoxyphenyl)ethyl)-D(oder L)-glucose
2-Deoxy-2-(2-hydroxy-2-(4-methoxyphenyl)ethyl)-D(oder L)-galactose
2-Deoxy-2-(2-hydroxy-2-(4-methoxyphenyl)ethyl)-D(oder L)-maltose
2-Deoxy-2-(2-hydroxy-2-(4-methoxyphenyl)ethyl)-D(oder L)-mannose
2-Deoxy-2-(2-hydroxy-2-(4-methoxyphenyl)ethyl)-D(oder L)-xylose
2-Deoxy-2-(2-hydroxy-2-(4-methoxyphenyl)ethyl)-D(oder L)-fucose
2-Deoxy-2-(2-hydroxy-2-(4-methoxyphenyl)ethyl)-D(oder L)-lactose
2-Deoxy-2-(2-hydroxy-2-(4-methoxyphenyl)ethyl)-D(oder L)-rhamnose
2-Deoxy-2-(2-oxo-2-(4-hydroxyphenyl)ethyl)-D(oder L)-glucose
2-Deoxy-2-(2-oxo-2-(4-hydroxyphenyl)ethyl)-D(oder L)-galactose
2-Deoxy-2-(2-oxo-2-(4-hydroxyphenyl)ethyl)-D(oder L)-maltose
2-Deoxy-2-(2-oxo-2-(4-hydroxyphenyl)ethyl)-D(oder L)-mannose
2-Deoxy-2-(2-oxo-2-(4-hydroxyphenyl)ethyl)-D(oder L)-xylose
2-Deoxy-2-(2-oxo-2-(4-hydroxyphenyl)ethyl)-D(oder L)-fucose
2-Deoxy-2-(2-oxo-2-(4-hydroxyphenyl)ethyl)-D(oder L)-lactose
2-Deoxy-2-(2-oxo-2-(4-hydroxyphenyl)ethyl)-D(oder L)-rhamnose
2-Deoxy-2-(2-oxo-2-(4-methoxyphenyl)ethyl)-D(oder L)-glucose
2-Deoxy-2-(2-oxo-2-(4-methoxyphenyl)ethyl)-D(oder L)-galactose
2-Deoxy-2-(2-oxo-2-(4-methoxyphenyl)ethyl)-D(oder L)-maltose
2-Deoxy-2-(2-oxo-2-(4-methoxyphenyl)ethyl)-D(oder L)-mannose
2-Deoxy-2-(2-oxo-2-(4-methoxyphenyl)ethyl)-D(oder L)-xylose
2-Deoxy-2-(2-oxo-2-(4-methoxyphenyl)ethyl)-D(oder L)-fucose
2-Deoxy-2-(2-oxo-2-(4-methoxyphenyl)ethyl)-D(oder L)-lactose
2-Deoxy-2-(2-oxo-2-(4-methoxyphenyl)ethyl)-D(oder L)-rhamnose
2-Deoxy-2-(2-hydroxy-2-(3-methoxy-4-hydroxyphenyl)ethyl)-D(oder L)-glucose
2-Deoxy-2-(2-hydroxy-2-(3-methoxy-4-hydroxyphenyl)ethyl)-D(oder L)-galactose
2-Deoxy-2-(2-hydroxy-2-(3-methoxy-4-hydroxyphenyl)ethyl)-D(oder L)-maltose
2-Deoxy-2-(2-hydroxy-2-(3-methoxy-4-hydroxyphenyl)ethyl)-D(oder L)-mannose
2-Deoxy-2-(2-hydroxy-2-(3-methoxy-4-hydroxyphenyl)ethyl)-D(oder L)-xylose
2-Deoxy-2-(2-hydroxy-2-(3-methoxy-4-hydroxyphenyl)ethyl)-D(oder L)-fucose
2-Deoxy-2-(2-hydroxy-2-(3-methoxy-4-hydroxyphenyl)ethyl)-D(oder L)-lactose
2-Deoxy-2-(2-hydroxy-2-(3-methoxy-4-hydroxyphenyl)ethyl)-D(oder L)-rhamnose
2-Deoxy-2-(2-(4-hydroxyphenyl)ethyl)-D(oder L)-glucose
2-Deoxy-2-(2-(4-hydroxyphenyl)ethyl)-D(oder L)-galactose
2-Deoxy-2-(2-(4-hydroxyphenyl)ethyl)-D(oder L)-maltose
2-Deoxy-2-(2-(4-hydroxyphenyl)ethyl)-D(oder L)-mannose
2-Deoxy-2-(2-(4-hydroxyphenyl)ethyl)-D(oder L)-xylose
2-Deoxy-2-(2-(4-hydroxyphenyl)ethyl)-D(oder L)-fucose
2-Deoxy-2-(2-(4-hydroxyphenyl)ethyl)-D(oder L)-lactose
2-Deoxy-2-(2-(4-hydroxyphenyl)ethyl)-D(oder L)-rhamnose
2-Deoxy-2-(2-methylamino-2-(4-hydroxyphenyl)ethyl)-D(oder L)-glucose
2-Deoxy-2-(2-methylamino-2-(4-hydroxyphenyl)ethyl)-D(oder L)-galactose
2-Deoxy-2-(2-methylamino-2-(4-hydroxyphenyl)ethyl)-D(oder L)-maltose
2-Deoxy-2-(2-methylamino-2-(4-hydroxyphenyl)ethyl)-D(oder L)-mannose
2-Deoxy-2-(2-methylamino-2-(4-hydroxyphenyl)ethyl)-D(oder L)-xylose
2-Deoxy-2-(2-methylamino-2-(4-hydroxyphenyl)ethyl)-D(oder L)-fucose
2-Deoxy-2-(2-methylamino-2-(4-hydroxyphenyl)ethyl)-D(oder L)-lactose
2-Deoxy-2-(2-methylamino-2-(4-hydroxyphenyl)ethyl)-D(oder L)-rhamnose
2-Deoxy-2-(2-amino-2-(4-hydroxyphenyl)ethyl)-D(oder L)-glucose
2-Deoxy-2-(2-amino2-(4-hydroxyphenyl)ethyl)-D(oder L)-galactose
2-Deoxy-2-(2-amino-2-(4-hydroxyphenyl)ethyl)-D(oder L)-maltose
2-Deoxy-2-(2-amino-2-(4-hydroxyphenyl)ethyl)-D(oder L)-mannose
2-Deoxy-2-(2-amino-2-(4-hydroxyphenyl)ethyl)-D(oder L)-xylose
2-Deoxy-2-(2-amino-2-(4-hydroxyphenyl)ethyl)-D(oder L)-fucose
2-Deoxy-2-(2-amino-2-(4-hydroxyphenyl)ethyl)-D(oder L)-lactose
2-Deoxy-2-(2-amino-2-(4-hydroxyphenyl)ethyl)-D(oder L)-rhamnose
2-Deoxy-2-(2-hydroxy-2-(4-hydroxyphenyl)ethyl)-D(oder L)-lyxose
2-Deoxy-2-(2-hydroxy-2-(4-methoxyphenyl)ethyl)-D(oder L)-lyxose
2-Deoxy-2-(2-oxo-2-(4-hydroxyphenyl)ethyl)-D(oder L)-lyxose
2-Deoxy-2-(2-oxo-2-(4-methoxyphenyl)ethyl)-D(oder L)-lyxose
2-Deoxy-2-(2-hydroxy-2-(3-methoxy-4-hydroxyphenyl)ethyl)-D(oder L)-lyxose
2-Deoxy-2-(2-(4-hydroxyphenyl)ethyl)-D(oder L)-lyxose
2-Deoxy-2-(2-methylamino-2-(4-hydroxyphenyl)ethyl)-D(oder L)-lyxose
2-Deoxy-2-(2-amino-2-(4-hydroxyphenyl)ethyl)-D(oder L)-lyxose
2-Deoxy-2-(2-hydroxy-2-(4-hydroxyphenyl)ethyl)-D(oder L)-arabinose
2-Deoxy-2-(2-hydroxy-2-(4-methoxyphenyl)ethyl)-D(oder L)-arabinose
2-Deoxy-2-(2-oxo-2-(4-hydroxyphenyl)ethyl)-D(oder L)-arabinose
2-Deoxy-2-(2-oxo-2-(4-methoxyphenyl)ethyl)-D(oder L)-arabinose
2-Deoxy-2-(2-hydroxy-2-(3-methoxy-4-hydroxyphenyl)ethyl)-D(oder L)-arabinose
2-Deoxy-2-(2-(4-hydroxyphenyl)ethyl)-D(oder L)-arabinose
2-Deoxy-2-(2-methylamino-2-(4-hydroxyphenyl)ethyl)-D(oder L)-arabinose
2-Deoxy-2-(2-amino-2-(4-hydroxyphenyl)ethyl)-D(oder L)-arabinose
2-Deoxy-2-(2-hydroxy-2-(4-hydroxyphenyl)ethyl)-D(oder L)-N-acetylglucosamin
2-Deoxy-2-(2-hydroxy-2-(4-methoxyphenyl)ethyl)-D(oder L)-N-acetylglucosamin
2-Deoxy-2-(2-oxo-2-(4-hydroxyphenyl)ethyl)-D(oder L)-N-acetylglucosamin
2-Deoxy-2-(2-oxo-2-(4-methoxyphenyl)ethyl)-D(oder L)-N-acetylglucosamin
2-Deoxy-2-(2-hydroxy-2-(3-methoxy-4-hydroxyphenyl)ethyl)-D(oder L)-N-acetylglucosamin
2-Deoxy-2-(2-(4-hydroxyphenyl)ethyl)-D(oder L)-N-acetylglucosamin
2-Deoxy-2-(2-methylamino-2-(4-hydroxyphenyl)ethyl)-D(oder L)-N-acetylglucosamin
2-Deoxy-2-(2-amino-2-(4-hydroxyphenyl)ethyl)-D(oder L)-N-acetylglucosamin
2-Deoxy-2-(2-hydroxy-2-(4-hydroxyphenyl)ethyl)-D(oder L)-N-acetylgalactosamin
2-Deoxy-2-(2-hydroxy-2-(4-methoxyphenyl)ethyl)-D(oder L)-N-acetylgalactosamin
2-Deoxy-2-(2-oxo-2-(4-hydroxyphenyl)ethyl)-D(oder L)-N-acetylgalactosamin
2-Deoxy-2-(2-oxo-2-(4-methoxyphenyl)ethyl)-D(oder L)-N-acetylgalactosamin
2-Deoxy-2-(2-hydroxy-2-(3-methoxy-4-hydroxyphenyl)ethyl)-D(oder L)-N-acetylgalactosamin
2-Deoxy-2-(2-(4-hydroxyphenyl)ethyl)-D(oder L)-N-acetylgalactosamin
2-Deoxy-2-(2-methylamino-2-(4-hydroxyphenyl)ethyl)-D(oder L)-N-acetylgalactosamin
2-Deoxy-2-(2-amino-2-(4-hydroxyphenyl)ethyl)-D(oder L)-N-acetylgalactosamin.

27. C-Glycosidverbindungen nach einem der Ansprüche 17 bis 26, **dadurch gekennzeichnet, dass** die Verbindung der Formel (I') ausgewählt ist unter:
2-Deoxy-2-(2-oxo-2-(4-hydroxyphenyl)ethyl)-D(oder L)-glucose
2-Deoxy-2-(2-hydroxy-2-(4-hydroxyphenyl)ethyl)-D(oder L)-glucose
2-Deoxy-2-(2-oxo-2-(4-methoxyphenyl)ethyl)-D(oder L)-glucose
2-Deoxy-2-(2-hydroxy-2-(4-methoxyphenyl)ethyl)-D(oder L)-glucose.

## Revendications

1. Composition comprenant, dans un milieu physiologiquement acceptable, au moins un composé de formule (I) : 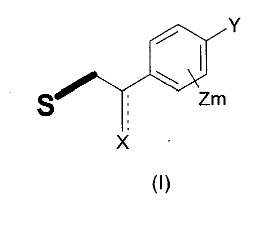 dans laquelle :
- S représente un monosaccharide ou un polysaccharide ayant jusqu'à 20 unités sucres, sous forme pyranose et/ou furanose et de série L et/ou D, ledit mono- ou polysaccharide présentant au moins une fonction hydroxyle obligatoirement libre et/ou éventuellement une ou plusieurs fonctions amine éventuellement protégée ;
- la liaison S-CH₂ étant une liaison C-glycosidique ;
- X représente un atome d'hydrogène ou un groupement choisi parmi -OR₁, -NR₁R₂ ou encore un groupement =O ;
- Y représente un groupement choisi parmi -OR₃, -NR₁R₂, -OSi(R₄)₃ ;
- Z représente un atome de fluor ou un groupement choisi parmi un groupe alkyle linéaire en C₁-C₆, un groupe alkyle cyclique ou ramifié en C₃-C₆, -OR₁, -NR₁R₂, - COR₁, -CO₂R₁, -CONR₁R₂, -CF₃ ;
- m est un nombre entier allant de 0 à 4 ;
avec
- R₁ et R₂ désignant, indépendamment l'un de l'autre, un atome d'hydrogène, ou un groupe alkyl linéaire en C₁-C₆, un groupe alkyle cyclique ou ramifié en C₃-C₆, aralkyle, phényle ;
- R₃ désignant un atome d'hydrogène, ou un groupe alkyl linéaire en C₁-C₆, un groupe alkyle cyclique ou ramifié en C₃-C₆, aralkyle, phényle, ou un groupement - SO₃H, -PO₃H₂, -COR₁ ;
- R₄ désignant un groupe alkyle linéaire en C₁-C₆, un groupe alkyle cyclique ou ramifié en C₃-C₆, phényle ;
ainsi que leurs sels cosmétiquement acceptables, leurs solvates tels que les hydrates ;
et un constituant choisi parmi l'eau, les huiles d'origine minérale, animale et/ou végétale, les cires, les pigments, les charges, les tensioactifs, les filtres UV, les polymères, les gélifiants.

2. Composition selon la revendication précédente, **caractérisée par le fait que** S représente un monosaccharide ou disaccharide dont la fonction hydroxyle en position 3 est libre.

3. Composition selon l'une quelconque des revendications précédentes, **caractérisée par** la fait que S représente un monosaccharide choisi parmi le D-glucose, le D-galactose, le D-mannose, le D-fucose, le L-fucose, le D-xylose, le D-lyxose, l'acide D-glucuronique, l'acide D-galacturonique, l'acide D-iduronique, la N-acétyl-D-glucosamine, la N-acétyl- D-galactosamine.

4. Composition selon l'une quelconque des revendications précédentes, **caractérisée par** la fait que S représente le D-glucose.

5. Composition selon la revendication 1 ou 2, **caractérisée par le fait que** S représente un polysaccharide contenant jusqu'à 6 unités sucre.

6. Composition selon l'une quelconque des revendications 1, 2 et 5, **caractérisée par** le fait S représente un polysaccharide choisi parmi le D-maltose, le D-lactose, le D-cellobiose, le D-maltotriose, un disaccharide associant un acide uronique choisi parmi l'acide D-iduronique, l'acide D-glucuronique ou l'acide D-galacturonique avec une hexosamine choisi parmi la D-galactosamine, la D-glucosamine, la N-acétyl-D-galactosamine, la N-acétyl-D-glucosamine, un oligosaccharide contenant au moins un xylose.

7. Composition selon l'une quelconque des revendications 1, 2, 5, 6, **caractérisée par** le fait S représente un polysaccharide choisi parmi le D-maltose et le D-lactose.

8. Composition selon l'une quelconque des revendications précédentes, **caractérisée par** la fait que :
- X désigne un groupement choisi parmi =O, -OH, -NH₂, - NHCH₃ ;
- Z désigne un groupement choisi parmi -NH₂, -CH₃, -OH, -OCH₃ ;
- Y désigne un groupement -OR₃ tel que défini dans la revendication 1;
- m désigne un nombre entier égal à 0 ou 1 ou 2.

9. Composition selon l'une quelconque des revendications précédentes, **caractérisée par** la fait que :
- S désigne le résidu d'un saccharide choisi parmi le glucose, le mannose, le xylose, le fucose, le galactose, le maltose, le lactose, le rhamnose, le lyxose, l'arabinose, la N-acétyl glucosamine, la N-acétyl galactosamine ;
- X désigne un groupement choisi parmi =O, -OH, -NH₂, - NHCH₃ ;
- Y désigne un groupement choisi parmi -OPO₃H₂, -OSO₃H, -OAc, -OH, -OCH₃ ;
- m=0.

10. Composition selon l'une quelconque des revendications précédentes, **caractérisée par** la fait que le composé de formule (I) est choisi parmi :
2-déoxy-2-(2-hydroxy-2-(4-hydroxy-phényl)-éthyl)-D(ou L)-glucose
2-déoxy-2-(2-hydroxy-2-(4-hydroxy-phényl)-éthyl)-D(ou L)-galactose
2-déoxy-2-(2-hydroxy-2-(4-hydroxy-phényl)-éthyl)-D(ou L)-maltose
2-déoxy-2-(2-hydroxy-2-(4-hydroxy-phényl)-éthyl)-D(ou L)-mannose
2-déoxy-2-(2-hydroxy-2-(4-hydroxy-phényl)-éthyl)-D(ou L)-xylose
2-déoxy-2-(2-hydroxy-2-(4-hydroxy-phényl)-éthyl)-D(ou L)-fucose
2-déoxy-2-(2-hydroxy-2-(4-hydroxy-phényl)-éthyl)-D (ou L)-lactose
2-déoxy-2-(2-hydroxy-2-(4-hydroxy-phényl)-éthyl)-D (ou L)-rhamnose
2-déoxy-2-(2-hydroxy-2-(4-méthoxy-phényl)-éthyl)-D(ou L)-glucose
2-déoxy-2-(2-hydroxy-2-(4-méthoxy-phényl)-éthyl)-D(ou L)-galactose
2-déoxy-2-(2-hydroxy-2-(4-méthoxy-phényl)-éthyl)-D(ou L)-maltose
2-déoxy-2-(2-hydroxy-2-(4-méthoxy-phényl)-éthyl)-D(ou L)-mannose
2-déoxy-2-(2-hydroxy-2-(4-méthoxy-phényl)-éthyl)-D(ou L)-xylose
2-déoxy-2-(2-hydroxy-2-(4-méthoxy phényl)-éthyl)-D(ou L)-fucose
2-déoxy-2-(2-hydroxy-2-(4-méthoxy-phényl)-éthyl)-D (ou L)-lactose
2-déoxy-2-(2-hydroxy-2-(4-méthoxy-phényl)-éthyl)-D (ou L)-rhamnose
2-déoxy-2-(2-oxo-2-(4-hydroxy-phényl)-éthyl)-D(ou L)-glucose
2-déoxy-2-(2-oxo-2-(4-hydroxy-phényl)-éthyl)-D(ou L)-galactose
2-déoxy-2-(2-oxo-2-(4-hydroxy-phényl)-éthyl)-D(ou L)-maltose
2-déoxy-2-(2-oxo-2-(4-hydroxy-phényl)-éthyl)-D(ou L)-mannose
2-déoxy-2-(2-oxo-2-(4-hydroxy-phényl)-éthyl)-D(ou L)-xylose
2-déoxy-2-(2-oxo-2-(4-hydroxy-phényl)-éthyl)-D(ou L)-fucose
2-déoxy-2-(2-oxo-2-(4-hydroxy-phényl)-éthyl)-D (ou L)-lactose
2-déoxy-2-(2-oxo-2-(4-hydroxy-phényl)-éthyl)-D (ou L)-rhamnose
2-déoxy-2-(2-oxo-2-(4-méthoxy-phényl)-éthyl)-D(ou L)-glucose 2-déoxy-2-(2-oxo-2-(4-méthoxy-phényl)-éthyl)-D(ou L)-galactose
2-déoxy-2-(2-oxo-2-(4-méthoxy-phényl)-éthyl)-D(ou L)-maltose
2-déoxy-2-(2-oxo-2-(4-méthoxy-phényl)-éthyl)-D(ou L)-mannose
2-déoxy-2-(2-oxo-2-(4-methooxy-phényl)-éthyl)-D(ou L)-xylose
2-déoxy-2-(2-oxo-2-(4-méthoxy-phényl)-éthyl)-D(ou L)-fucose
2-déoxy-2-(2-oxo-2-(4-méthoxy-phényl)-éthyl)-D (ou L)-lactose
2-déoxy-2-(2-oxo-2-(4-méthoxy-phényl)-éthyl)-D (ou L)-rhamnose
2-déoxy-2-(2-hydroxy-2-(3-méthoxy-4-hydroxy-phényl)-éthyl)-D(ou L)-glucose
2-déoxy-2-(2-hydroxy-2-(3-méthoxy-4-hydroxy-phényl)-éthyl)-D(ou L)-galactose
2-déoxy-2-(2-hydroxy-2-(3-méthoxy-4-hydroxy-phényl)-éthyl)-D(ou L)-maltose
2-déoxy-2-(2-hydroxy-2-(3-méthoxy-4-hydroxy-phényl)-éthyl)-D(ou L)-mannose
2-déoxy-2-(2-hydroxy-2-(3-méthoxy-4-hydroxy-phényl)-éthyl)-D(ou L)-xylose
2-déoxy-2-(2-hydroxy-2-(3-méthoxy-4-hydroxy-phényl)-éthyl)-D(ou L)-fucose
2-déoxy-2-(2-hydroxy-2-(3-méthoxy-4-hydroxy-phényl)-éthyl)-D (ou L)-lactose
2-déoxy-2-(2-hydroxy-2-(3-méthoxy-4-hydroxy-phényl)-éthyl)-D (ou L)-rhamnose
2-déoxy-2-(2-(4-hydroxy-phényl)-éthyl)-D(ou L)-glucose
2-déoxy-2-(2-(4-hydroxy-phényl)-éthyl)-D(ou L)-galactose
2-déoxy-2-(2-(4-hydroxy-phényl)-éthyl)-D(ou L)-maltose
2-déoxy-2-(2-(4-hydroxy-phényl)-éthyl)-D(ou L)-mannose
2-déoxy-2-(2-(4-hydroxy-phényl)-éthyl)-D(ou L)-xylose
2-déoxy-2-(2-(4-hydroxy-phényl)-éthyl)-D(ou L)-fucose
2-déoxy-2-(2-(4-hydroxy-phényl)-éthyl)-D (ou L)-lactose
2-déoxy-2-(2-(4-hydroxy-phényl)-éthyl)-D (ou L)-rhamnose
2-déoxy-2-(2-méthylamino-2-(4-hydroxy-phényl)-éthyl)-D(ou L)-glucose
2-déoxy-2-(2-méthylamino-2-(4-hydroxy-phényl)-éthyl)-D(ou L)-galactose
2-déoxy-2-(2-méthylamino-2-(4-hydroxy-phényl)-éthyl)-D(ou L)-maltose
2-déoxy-2-(2-méthylamino-2-(4-hydroxy-phényl)-éthyl)-D(ou L)-mannose
2-déoxy-2-(2-méthylamino-2-(4-hydroxy-phényl)-éthyl)-D(ou L)-xylose
2-déoxy-2-(2-méthylamino-2-(4-hydroxy-phényl)-éthyl)-D(ou L)-fucose
2-déoxy-2-(2-méthylamino-2-(4-hydroxy-phényl)-éthyl)-D (ou L)-lactose
2-déoxy-2-(2-méthylamino-2-(4-hydroxy-phényl)-éthyl)-D (ou L)-rhamnose
2-déoxy-2-(2-amino-2-(4-hydroxy-phényl)-éthyl)-D(ou L)-glucose
2-déoxy-2-(2-amino-2-(4-hydroxy-phényl)-éthyl)-D(ou L)-galactose
2-déoxy-2-(2-amino-2-(4-hydroxy-phényl)-éthyl)-D(ou L)-maltose
2-déoxy-2-(2-amino-2-(4-hydroxy-phényl)-éthyl)-D(ou L)-mannose
2-déoxy-2-(2-amino-2-(4-hydroxy-phényl)-éthyl)-D(ou L)-xylose
2-déoxy-2-(2-amino-2-(4-hydroxy-phényl)-éthyl)-D(ou L)-fucose
2-déoxy-2-(2-amino-2-(4-hydroxy-phényl)-éthyl)-D (ou L)-lactose
2-déoxy-2-(2-amino-2-(4-hydroxy-phényl)-éthyl)-D (ou L)-rhamnose
2-déoxy-2-(2-hydroxy-2-(4-hydroxy-phényl)-éthyl)-D (ou L)-lyxose
2-déoxy-2-(2-hydroxy-2-(4-méthoxy-phényl)-éthyl)-D (ou L)- lyxose
2-déoxy-2-(2-oxo-2-(4-hydroxy-phényl)-éthyl)-D (ou L)- lyxose
2-déoxy-2-(2-oxo-2-(4-méthoxy-phényl)-éthyl)-D (ou L)- lyxose
2-déoxy-2-(2-hydroxy-2-(3-méthoxy-4-hydroxy-phényl)-éthyl)-D (ou L)- lyxose
2-déoxy-2-(2-(4-hydroxy-phényl)-éthyl)-D (ou L)- lyxose
2-déoxy-2-(2-méthylamino-2-(4-hydroxy-phényl)-éthyl)-D (ou L)- lyxose
2-déoxy-2-(2-amino-2-(4-hydroxy-phényl)-éthyl)-D (ou L)- lyxose
2-déoxy-2-(2-hydroxy-2-(4-hydroxy-phényl)-éthyl)-D (ou L)-arabinose
2-déoxy-2-(2-hydroxy-2-(4-méthoxy-phényl)-éthyl)-D (ou L)- arabinose
2-déoxy-2-(2-oxo-2-(4-hydroxy-phényl)-éthyl)-D (ou L)- arabinose
2-déoxy-2-(2-oxo-2-(4-méthoxy-phényl)-éthyl)-D (ou L)- arabinose
2-déoxy-2-(2-hydroxy-2-(3-méthoxy-4-hydroxy-phényl)-éthyl)-D (ou L)- arabinose
2-déoxy-2-(2-(4-hydroxy-phényl)-éthyl)-D (ou L)- arabinose
2-déoxy-2-(2-méthylamino-2-(4-hydroxy-phényl)-éthyl)-D (ou L)- arabinose
2-déoxy-2-(2-amino-2-(4-hydroxy-phényl)-éthyl)-D (ou L)- arabinose
2-déoxy-2-(2-hydroxy-2-(4-hydroxy-phényl)-éthyl)-D (ou L)- N-acétyl glucosamine
2-déoxy-2-(2-hydroxy-2-(4-méthoxy-phényl)-éthyl)-D (ou L)- N-acétyl glucosamine
2-déoxy-2-(2-oxo-2-(4-hydroxy-phényl)-éthyl)-D (ou L)- N-acétyl glucosamine
2-déoxy-2-(2-oxo-2-(4-méthoxy-phényl)-éthyl)-D (ou L)- N-acétyl glucosamine
2-déoxy-2-(2-hydroxy-2-(3-méthoxy-4-hydroxy-phényl)-éthyl)-D (ou L)- N-acétyl glucosamine
2-déoxy-2-(2-(4-hydroxy-phényl)-éthyl)-D (ou L)- N-acétyl glucosamine
2-déoxy-2-(2-méthylamino-2-(4-hydroxy-phényl)-éthyl)-D (ou L)- N-acétyl glucosamine
2-déoxy-2-(2-amino-2-(4-hydroxy-phényl)-éthyl)-D (ou L)- N-acétyl glucosamine
72-déoxy-2-(2-hydroxy-2-(4-hydroxy-phényl)-éthyl)-D (ou L)- N-acétyl galactosamine
2-déoxy-2-(2-hydroxy-2-(4-méthoxy-phényl)-éthyl)-D (ou L)- N-acétyl galactosamine
2-déoxy-2-(2-oxo-2-(4-hydroxy-phényl)-éthyl)-D (ou L)- N-acétyl galactosamine
2-déoxy-2-(2-oxo-2-(4-méthoxy-phényl)-éthyl)-D (ou L)- N-acétyl galactosamine
2-déoxy-2-(2-hydroxy-2-(3-méthoxy-4-hydroxy-phényl)-éthyl)-D (ou L)- N-acétyl galactosamine
2-déoxy-2-(2-(4-hydroxy-phényl)-éthyl)-D (ou L)- N-acétyl galactosamine
2-déoxy-2-(2-méthylamino-2-(4-hydroxy-phényl)-éthyl)-D (ou L)- N-acétyl galactosamine
2-déoxy-2-(2-amino-2-(4-hydroxy-phényl)-éthyl)-D (ou L)- N-acétyl galactosamine

11. Composition selon l'une quelconque des revendications précédentes, **caractérisée par** la fait que le composé de formule (I) est choisi parmi :
le 2-déoxy-2-(2-oxo-2-(4-hydroxy-phényl)-éthyl)-D(ou L)-glucose ;
le 2-déoxy-2-(2-hydroxy-2-(4-hydroxy-phényl)-éthyl)-D(ou L)-glucose ;
le 2-déoxy-2-(2-oxo-2-(4-méthoxy-phényl)-éthyl)-D(ou L)-glucose ;
le 2-déoxy-2-(2-hydroxy-2-(4-méthoxy-phényl)-éthyl)-D(ou L)-glucose.

12. Composition selon l'une quelconque des revendications précédentes, **caractérisée par** la fait que le composé de formule (I) est présent en une quantité allant de 0,001% à 10% en poids, par rapport au poids total de la composition, de préférence allant de 0,01 % à 5% en poids.

13. Composition selon l'une quelconque des revendications précédentes, **caractérisée qu'**elle est destinée à dépigmenter et/ou blanchir la peau.

14. Procédé cosmétique non thérapeutique de dépigmentation et/ou de blanchiment de la peau humaine, des poils ou des cheveux comprenant l'application sur la peau, les poils ou les cheveux d'une composition selon l'une quelconque des revendications précédentes.

15. Utilisation cosmétique non thérapeutique d'un composé de formule (I) tel que défini selon l'une quelconque des revendications 1 à 11, comme agent blanchissant et/ou dépigmentant de la peau.

16. Utilisation d'un composé de formule (I) tel que défini selon l'une quelconque des revendications 1 à 11, pour la fabrication d'une composition dermatologique destinée à dépigmenter et/ou blanchir la peau.

17. Composés C-glycosides de formule (I') : 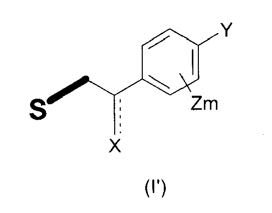 dans laquelle :
- S représente un monosaccharide ou un polysaccharide ayant jusqu'à 20 unités sucres, sous forme pyranose et/ou furanose et de série L et/ou D, ledit mono- ou polysaccharide présentant au moins une fonction hydroxyle obligatoirement libre et/ou éventuellement une ou plusieurs fonctions amine éventuellement protégée ;
- la liaison S-CH₂ étant une liaison C-glycosidique ;
- X représente un atome d'hydrogène ou un groupement choisi parmi -OR₁,
- NR₁R₂ ou encore un groupement =O ;
- Y représente un groupement choisi parmi -OR₃, -NR₁R₂, -OSi(R₄)₃ ;
- Z représente un atome de fluor ou un groupement choisi parmi un groupe alkyle linéaire en C₁-C₆, un groupe alkyle cyclique ou ramifié en C₃-C₆, -OR₁, -NR₁R₂, - COR₁, -CO₂R₁, -CONR₁R₂, -CF₃ ;
- m est un nombre entier allant de 0 à 4 ;
avec
- R₁ et R₂ désignant, indépendamment l'un de l'autre, un atome d'hydrogène, ou un groupe alkyl linéaire en C₁-C₆, un groupe alkyle cyclique ou ramifié en C₃-C₆, aralkyle, phényle ;
- R₃ désignant un atome d'hydrogène, ou un groupe alkyl linéaire en C₁-C₆, un groupe alkyle cyclique ou ramifié en C₃-C₆, aralkyle, phényle, ou un groupement - SO₃H, -PO₃H₂, -COR₁;
- R₄ désignant un groupe alkyle linéaire en C₁-C₆, un groupe alkyle cyclique ou ramifié en C₃-C₆, phényle ;
sous réserve que lorsque m = 0 et Y désigne un groupement -OCH₃, S ne désigne pas un résidu D (ou L)-glucose, L-fucose, D (ou L)-xylose, D (ou L)-maltose,
ainsi que leurs sels cosmétiquement acceptables, leurs solvates tels que les hydrates.

18. Composés C-glycosides selon la revendication précédente, **caractérisés par le fait que** S représente un monosaccharide ou disaccharide dont la fonction hydroxyle en position 3 est libre.

19. Composés C-glycosides selon la revendication 17 ou 18, **caractérisés par le fait que** S représente un monosaccharide choisi parmi le D-glucose, le D-galactose, le D-mannose, le D-fucose, le L-fucose, le D-xylose, le D-lyxose, l'acide D-glucuronique, l'acide D-galacturonique, l'acide D-iduronique, la N-acétyl-D-glucosamine, la N-acétyl- D-galactosamine.

20. Composés C-glycosides selon l'une quelconque des revendications 17 à 19, **caractérisés par le fait que** S représente le D-glucose.

21. Composés C-glycosides selon la revendication 17 ou 18, **caractérisés par le fait que** S représente un polysaccharide contenant jusqu'à 6 unités sucre.

22. Composés C-glycosides selon la revendication précédente, **caractérisés par le fait que** S représente un polysacchride choisi parmi le D-maltose, le D-lactose, le D-cellobiose, le D-maltotriose, un disaccharide associant un acide uronique choisi parmi l'acide D-iduronique, l'acide D-glucuronique ou l'acide D-galacturonique avec une hexosamine choisi parmi la D-galactosamine, la D-glucosamine, la N-acétyl-D-galactosamine, la N-acétyl-D-glucosamine, un oligosaccharide contenant au moins un xylose.

23. Composés C-glycosides selon la revendication 21 ou 22, **caractérisés par le fait que** S représente un polysaccharide choisi parmi le D-maltose et le D-lactose.

24. Composés C-glycosides selon l'une quelconque des revendications 17 à 23, **caractérisés par le fait que** :
- X désigne un groupement choisi parmi =O, -OH, -NH₂, - NHCH₃ ;
- Z désigne un groupement choisi parmi -NH₂, -CH₃, -OH, -OCH₃ ;
- Y désigne un groupement -OR₃ tel que défini dans la revendication 17;
- m désigne un nombre entier égal à 0 ou 1 ou 2.

25. Composés C-glycosides selon l'une quelconque des revendications 17 à 24, **caractérisés par le fait que** :
- S désigne le résidu d'un saccharide choisi parmi le glucose, le mannose, le xylose, le fucose, le galactose, le maltose, le lactose, le rhamnose, le lyxose, l'arabinose, la N-acétyl glucosamine, la N-acétyl galactosamine ;
- X désigne un groupement choisi parmi =O, -OH, -NH₂, - NHCH₃ ;
- Y désigne un groupement choisi parmi -OPO₃H₂, -OSO₃H, -OAc, -OH, -OCH₃ ;
- m = 0.

26. Composés C-glycosides selon l'une quelconque des revendications 17 à 25, **caractérisés par le fait que** :le composé de formule (I') est choisi parmi :
2-déoxy-2-(2-hydroxy-2-(4-hydroxy-phényl)-éthyl)-D(ou L)-glucose
2-déoxy-2-(2-hydroxy-2-(4-hydroxy-phényl)-éthyl)-D(ou L)-galactose
2-déoxy-2-(2-hydroxy-2-(4-hydroxy-phényl)-éthyl)-D(ou L)-maltose
2-déoxy-2-(2-hydroxy-2-(4-hydroxy-phényl)-éthyl)-D(ou L)-mannose
2-déoxy-2-(2-hydroxy-2-(4-hydroxy-phényl)-éthyl)-D(ou L)-xylose
2-déoxy-2-(2-hydroxy-2-(4-hydroxy-phényl)-éthyl)-D(ou L)-fucose
2-déoxy-2-(2-hydroxy-2-(4-hydroxy-phényl)-éthyl)-D (ou L)-lactose
2-déoxy-2-(2-hydroxy-2-(4-hydroxy-phényl)-éthyl)-D (ou L)-rhamnose
2-déoxy-2-(2-hydroxy-2-(4-méthoxy-phényl)-éthyl)-D(ou L)-glucose
2-déoxy-2-(2-hydroxy-2-(4-méthoxy-phényl)-éthyl)-D(où L)-galactose
2-déoxy-2-(2-hydroxy-2-(4-méthoxy-phényl)-éthyl)-D(ou L)-maltose
2-déoxy-2-(2-hydroxy-2-(4-méthoxy-phényl)-éthyl)-D(ou L)-mannose
2-déoxy-2-(2-hydroxy-2-(4-méthoxy-phényl)-éthyl)-D(ou L)-xylose
2-déoxy-2-(2-hydroxy-2-(4-méthoxyphényl)-éthyl)-D(ou L)-fucose
2-déoxy-2-(2-hydroxy-2-(4-méthoxy-phényl)-éthyl)-D (ou L)-lactose
2-déoxy-2-(2-hydroxy-2-(4-méthoxy-phényl)-éthyl)-D (ou L)-rhamnose
2-déoxy-2-(2-oxo-2-(4-hydroxy-phényl)-éthyl)-D(ou L)-glucose
2-déoxy-2-(2-oxo-2-(4-hydroxy-phényl)-éthyl)-D(ou L)-galactose
2-déoxy-2-(2-oxo-2-(4-hydroxy-phényl)-éthyl)-D(ou L)-maltose
2-déoxy-2-(2-oxo-2-(4-hydroxy-phényl)-éthyl)-D(ou L)-mannose
2-déoxy-2-(2-oxo-2-(4-hydroxy-phényl)-éthyl)-D(ou L)-xylose
2-déoxy-2-(2-oxo-2-(4-hydroxy-phényl)-éthyl)-D(ou L)-fucose
2-déoxy-2-(2-oxo-2-(4-hydroxy-phényl)-éthyl)-D (ou L)-lactose
2-déoxy-2-(2-oxo-2-(4-hydroxy-phényl)-éthyl)-D (ou L)-rhamnose
2-déoxy-2-(2-oxo-2-(4-méthoxy-phényl)-éthyl)-D(ou L)-glucose
2-déoxy-2-(2-oxo-2-(4=méthoxy-phényl)-éthyl)-D(ou L)-galactose
2-déoxy-2-(2-oxo-2-(4-méthoxy-phényl)-éthyl)-D(ou L)-maltose
2-déoxy-2-(2-oxo-2-(4-méthoxy-phényl)-éthyl)-D(ou L)-mannose
2-déoxy-2-(2-oxo-2-(4-methooxy-phényl)-éthyl)-D(ou L)-xylose
2-déoxy-2-(2-oxo-2-(4-méthoxy-phényl)-éthyl)-D(ou L)-fucose
2-déoxy-2-(2-oxo-2-(4-méthoxy-phényl)-éthyl)-D (ou L)-lactose
2-déoxy-2-(2-oxo-2-(4-méthoxy-phényl)-éthyl)-D (ou L)-rhamnose
2-déoxy-2-(2-hydroxy-2-(3-méthoxy-4-hydroxy-phényl)-éthyl)-D(ou L)-glucose
2-déoxy-2-(2-hydroxy-2-(3-méthoxy-4-hydroxy-phényl)-éthyl)-D(ou L)-galactose
2-déoxy-2-(2-hydroxy-2-(3-méthoxy-4-hydroxy-phényl)-éthyl)-D(ou L)-maltose
2-déoxy-2-(2-hydroxy-2-(3-méthoxy-4-hydroxy-phényl)-éthyl)-D(ou L)-mannose
2-déoxy-2-(2-hydroxy-2-(3-méthoxy-4-hydroxy-phényl)-éthyl)-D(ou L)-xylose
2-déoxy-2-(2-hydroxy-2-(3-méthoxy-4-hydroxy-phényl)-éthyl)-D(ou L)-fucose
2-déoxy-2-(2-hydroxy-2-(3-méthoxy-4-hydroxy-phényl)-éthyl)-D (ou L)-lactose
2-déoxy-2-(2-hydroxy-2-(3-méthoxy-4-hydroxy-phényl)-éthyl)-D (ou L)-rhamnose
2-déoxy-2-(2-(4-hydroxy-phényl)-éthyl)-D(ou L)-glucose
2-déoxy-2-(2-(4-hydroxy-phényl)-éthyl)-D(ou L)-galactose
2-déoxy-2-(2-(4-hydroxy-phényl)-éthyl)-D(ou L)-maltose
2-déoxy-2-(2-(4-hydroxy-phényl)-éthyl)-D(ou L)-mannose
2-déoxy-2-(2-(4-hydroxy-phényl)-éthyl)-D(ou L)-xylose
2-déoxy-2-(2-(4-hydroxy-phényl)-éthyl)-D(ou L)-fucose
2-déoxy-2-(2-(4-hydroxy-phényl)-éthyl)-D (ou L)-lactose
2-déoxy-2-(2-(4-hydroxy-phényl)-éthyl)-D (ou L)-rhamnose
2-déoxy-2-(2-méthylamino-2-(4-hydroxy-phényl)-éthyl)-D(ou L)-glucose
2-déoxy-2-(2-méthylamino-2-(4-hydroxy-phényl)-éthyl)-D(ou L)-galactose
2-déoxy-2-(2-méthylamino-2-(4-hydroxy-phényl)-éthyl)-D(ou L)-maltose
2-déoxy-2-(2-méthylamino-2-(4-hydroxy-phényl)-éthyl)-D(ou L)-mannose
2-déoxy-2-(2-méthylamino-2-(4-hydroxy-phényl)-éthyl)-D(ou L)-xylose
2-déoxy-2-(2-méthylamino-2-(4-hydroxy-phényl)-éthyl)-D(ou L)-fucose
2-déoxy-2-(2-méthylamino-2-(4-hydroxy-phényl)-éthyl)-D (ou L)-lactose
2-déoxy-2-(2-méthylamino-2-(4-hydroxy-phényl)-éthyl)-D (ou L)-rhamnose
2-déoxy-2-(2-amino-2-(4-hydroxy-phényl)-éthyl)-D(ou L)-glucose
2-déoxy-2-(2-amino-2-(4-hydroxy-phényl)-éthyl)-D(ou L)-galactose
2-déoxy-2-(2-amino-2-(4-hydroxy-phényl)-éthyl)-D(ou L)-maltose
2-déoxy-2-(2-amino-2-(4-hydroxy-phényl)-éthyl)-D(ou L)-mannose
2-déoxy-2-(2-amino-2-(4-hydroxy-phényl)-éthyl)-D(ou L)-xylose
2-déoxy-2-(2-amino-2-(4-hydroxy-phényl)-éthyl)-D(ou L)-fucose
2-déoxy-2-(2-amino-2-(4-hydroxy-phényl)-éthyl)-D (ou L)-lactose
2-déoxy-2-(2-amino-2-(4-hydroxy-phényl)-éthyl)-D (ou L)-rhamnose
2-déoxy-2-(2-hydroxy-2-(4-hydroxy-phényl)-éthyl)-D (ou L)-lyxose
2-déoxy-2-(2-hydroxy-2-(4-méthoxy-phényl)-éthyl)-D (ou L)- lyxose
2-déoxy-2-(2-oxo-2-(4-hydroxy-phényl)-éthyl)-D (ou L)- lyxose
2-déoxy-2-(2-oxo-2-(4-méthoxy-phényl)-éthyl)-D (ou L)- lyxose
2-déoxy-2-(2-hydroxy-2-(3-méthoxy-4-hydroxy-phényl)-éthyl)-D (ou L)- lyxose
2-déoxy-2-(2-(4-hydroxy-phényl)-éthyl)-D (ou L)- lyxose
2-déoxy-2-(2-méthylamino-2-(4-hydroxy-phényl)-éthyl)-D (ou L)- lyxose
2-déoxy-2-(2-amino-2-(4-hydroxy-phényl)-éthyl)-D (ou L)- lyxose
2-déoxy-2-(2-hydroxy-2-(4-hydroxy-phényl)-éthyl)-D (ou L)-arabinose
2-déoxy-2-(2-hydroxy-2-(4-méthoxy-phényl)-éthyl)-D (ou L)- arabinose
2-déoxy-2-(2-oxo-2-(4-hydroxy-phényl)-éthyl)-D (ou L)- arabinose
2-déoxy-2-(2-oxo-2-(4-méthoxy-phényl)-éthyl)-D (ou L)- arabinose
2-déoxy-2-(2-hydroxy-2-(3-méthoxy-4-hydroxy-phényl)-éthyl)-D (ou L)- arabinose
2-déoxy-2-(2-(4-hydroxy-phényl)-éthyl)-D (ou L)- arabinose
2-déoxy-2-(2-méthylamino-2-(4-hydroxy-phényl)-éthyl)-D (ou L)- arabinose
2-déoxy-2-(2-amino-2-(4-hydroxy-phényl)-éthyl)-D (ou L)- arabinose
2-déoxy-2-(2-hydroxy-2-(4-hydroxy-phényl)-éthyl)-D (ou L)- N-acétyl glucosamine
2-déoxy-2-(2-hydroxy-2-(4-méthoxy-phényl)-éthyl)-D (ou L)- N-acétyl glucosamine
2-déoxy-2-(2-oxo-2₋(4-hydroxy-phényl)-éthyl)-D (ou L)- N-acétyl glucosamine
2-déoxy-2-(2-oxo-2-(4-méthoxy-phényl)-éthyl)-D (ou L)- N-acétyl glucosamine
2-déoxy-2-(2-hydroxy-2-(3-méthoxy-4-hydroxy-phényl)-éthyl)-D (ou L)- N-acétyl glucosamine
2-déoxy-2-(2-(4-hydroxy-phényl)-éthyl)-D (ou L)- N-acétyl glucosamine
2-déoxy-2-(2-méthylamino-2-(4-hydroxy-phényl)-éthyl)-D (ou L)- N-acétyl glucosamine
2-déoxy-2-(2-amino-2-(4-hydroxy-phényl)-éthyl)-D (ou L)- N-acétyl glucosamine
72-déoxy-2-(2-hydroxy-2-(4-hydroxy-phényl)-éthyl)-D (ou L)- N-acétyl galactosamine
2-déoxy-2-(2-hydroxy-2-(4-méthoxy-phényl)-éthyl)-D (ou L)- N-acétyl galactosamine
2-déoxy-2-(2-oxo-2-(4-hydroxy-phényl)-éthyl)-D (ou L)- N-acétyl galactosamine
2-déoxy-2-(2-oxo-2-(4-méthoxy-phényl)-éthyl)-D (ou L)- N-acétyl galactosamine
2-déoxy-2-(2-hydroxy-2-(3-méthoxy-4-hydroxy-phényl)-éthyl)-D (ou L)- N-acétyl galactosamine
2-déoxy-2-(2-(4-hydroxy-phényl)-éthyl)-D (ou L)- N-acétyl galactosamine
2-déoxy-2-(2-méthylamino-2-(4-hydroxy-phényl)-éthyl)-D (ou L)- N-acétyl galactosamine
2-déoxy-2-(2-amino-2-(4-hydroxy-phényl)-éthyl)-D (ou L)- N-acétyl galactosamine

27. Composés C-glycosides selon l'une quelconque des revendications 17 à 26, **caractérisés par le fait que** le composé de formule (l') est choisi parmi :
le 2-déoxy-2-(2-oxo-2-(4-hydroxy-phényl)-éthyl)-D(ou L)-glucose ;
le 2-déoxy-2-(2-hydroxy-2-(4-hydroxy-phényl)-éthyl)-D(ou L)-glucose ;
le 2-déoxy-2-(2-oxo-2-(4-méthoxy-phényl)-éthyl)-D(ou L)-glucose ;
le 2-déoxy-2-(2-hydroxy-2-(4-méthoxy-phényl)-éthyl)-D(ou L)-glucose.
